# EUROPEAN PATENT SPECIFICATION

(11) **EP 2 850 195 B1**
(45) Date of publication and mention of the grant of the patent: **22.01.2020**
(21) Application number: 13790429.8
(22) Date of filing: 14.03.2013
(51) Int. Cl.: C12N 15/861

(54) **METHODS AND COMPOSITIONS FOR TREATING AMYLOID DEPOSITS**
VERFAHREN UND ZUSAMMENSETZUNGEN ZUR BEHANDLUNG VON AMYLOIDABLAGERUNGEN
PROCÉDÉS ET COMPOSITIONS DE TRAITEMENT DE DÉPÔTS AMYLOÏDES

(30) Priority: 18.05.2012 US 201261648801 P
(43) Date of publication of application: 25.03.2015
(73) Proprietor: University of Iowa Research Foundation, Iowa City, IA 52242 (US); The General Hospital Corporation, Boston, MA 02114 (US)
(72) Inventor: DAVIDSON, Beverly, L., Iowa City, Iowa 52242 (US); HYMAN, Bradley, T., Boston Massachusetts 02114 (US)
(74) Representative: PATERIS Patentanwälte PartmbB
(86) International application number: PCT/US2013/031725
(87) International publication number: WO 2013/172964

(56) References cited:
- WO-A1-99/16464
- US-A1- 2011 038 851
- US-B1- 6 468 524
- FENG XUAN ET AL: "Adeno-associated viral vector-mediated ApoE expression in Alzheimer's disease mice: low CNS immune response, long-term expression, and astrocyte specificity.", FRONTIERS IN BIOSCIENCE : A JOURNAL AND VIRTUAL LIBRARY 1 MAY 2004, vol. 9, 1 May 2004 (2004-05-01), pages 1540-1546, XP009186571, ISSN: 1093-9946
- E. HUDRY ET AL: "Gene Transfer of Human Apoe Isoforms Results in Differential Modulation of Amyloid Deposition and Neurotoxicity in Mouse Brain", SCIENCE TRANSLATIONAL MEDICINE, vol. 5, no. 212, 20 November 2013 (2013-11-20), pages 212ra161-212ra161, XP055219657, Washington, DC ISSN: 1946-6234, DOI: 10.1126/scitranslmed.3007000
- J.-C. Dodart ET AL: "Gene delivery of human apolipoprotein E alters brain A? burden in a mouse model of Alzheimer's disease", Proceedings of the National Academy of Sciences, vol. 102, no. 4, 25 January 2005 (2005-01-25), pages 1211-1216, XP055374325, US ISSN: 0027-8424, DOI: 10.1073/pnas.0409072102

## Description

### BACKGROUND

Gene transfer is now widely recognized as a powerful tool for analysis of biological events and disease processes at both the cellular and molecular level. More recently, the application of gene therapy for the treatment of human diseases, either inherited (*e.g*., ADA deficiency) or acquired (*e.g.*, cancer or infectious disease), has received considerable attention. With the advent of improved gene transfer techniques and the identification of an ever expanding library of defective gene-related diseases, gene therapy has rapidly evolved from a treatment theory to a practical reality.

Traditionally, gene therapy has been defined as a procedure in which an exogenous gene is introduced into the cells of a patient in order to correct an inborn genetic error. More recently, gene therapy has been broadly defined as the correction of a disease phenotype through the introduction of new genetic information into the affected organism. In *in vivo* gene therapy, a transferred gene is introduced into cells of the recipient organism *in situ* that is, within the recipient. *In vivo* gene therapy has been examined in animal models. The feasibility of direct gene transfer *in situ* into organs and tissues such as muscle, hematopoietic stem cells, the arterial wall, the nervous system, and lung has been reported. Direct injection of DNA into skeletal muscle, heart muscle and injection of DNA-lipid complexes into the vasculature also has been reported to yield a detectable expression level of the inserted gene product(s) *in vivo.*

Treatment of diseases of the central nervous system (CNS), *e.g.,* genetic diseases of the brain such as Alzheimer's disease, remains an intractable problem. A major problem with treating brain diseases is that therapeutic proteins when delivered intravenously do not cross the blood-brain barrier, or when delivered directly to the brain, are not widely distributed. Thus, therapies for treating Alzheimer's disease need to be developed.

Dodart et al., PNAS, vol. 102, no. 4 (2005), pages 1211-1216 describes gene delivery of human apolipoprotein E to the brain by using a lentiviral vector in a mouse model of Alzheimer's disease. It also describes that apoE2 reduces brain Aβ burden in the mouse model compared to apoE3 and apoE4.

### SUMMARY

The present invention relates to an rAAV2 particle comprising an AAV2 capsid protein and a vector comprising a nucleic acid encoding a protective ApoE ε2 isoform protein inserted between a pair of AAV2 inverted terminal repeats, for use in treating Alzheimer's disease in a mammal, wherein the rAAV particle is administered to an ependymal cell thereby delivering the nucleic acid to the ependymal cell, wherein the ependymal cell secretes the ApoE ε2 protein so as to treat the disease, and wherein the mammal is a non-rodent mammal.

The present invention further relates to a pharmaceutical composition comprising an rAAV2 particle comprising an AAV2 capsid protein and a vector comprising a nucleic acid encoding a protective ApoE ε2 isoform protein inserted between a pair of AAV2 inverted terminal repeats, for use in treating Alzheimer's disease in a mammal, wherein the rAAV2 particle is administered to an ependymal cell thereby delivering the nucleic acid to the ependymal cell, wherein the ependymal cell secretes the ApoE ε2 protein so as to treat the disease, and wherein the mammal is a non-rodent mammal.

In certain embodiments, disclosed is a method of treating Alzheimer's disease in a mammal comprising administering to the cerebrospinal fluid (CSF) of the mammal an rAAV particle comprising an AAV capsid protein and a vector comprising a nucleic acid encoding a protective ApoE isoform protein inserted between a pair of AAV inverted terminal repeats in a manner effective to infect an ependymal cell in the non-rodent mammal, wherein the ependymal cell secretes the ApoE so as to treat the disease. As used herein, the term "protective ApoE isoform" is used to distinguish ApoE isoforms that decrease the risk of Alzheimer's disease by at least 5%, such as 10%, 20%, 30%, 40%, 50%, 60%, 70%, 80%, 90%, 100% or more.

In certain embodiments, disclosed is a method of delivering a protective ApoE isoform to the central nervous system of a non-rodent mammal, comprising administering to the cerebrospinal fluid (CSF) of the non-rodent mammal an rAAV particle comprising an AAV capsid protein and a vector comprising a nucleic acid encoding the protective ApoE isoform inserted between a pair of AAV inverted terminal repeats in a manner effective to infect ependymal cells in the non-rodent mammal such that the ependymal cells secrete the ApoE into the CSF of the mammal. In certain embodiments, the rAAV particle is an rAAV2 particle that infects the non-rodent ependymal cell at an rate of more than 20% than the infectivity rate of AAV4, such as at a rate of more than 50% or 100%, 1000% or 2000% than the infectivity rate of AAV4.

In certain embodiments, disclosed is a method of treating a disease in a non-rodent mammal comprising administering to ependymal cells of the mammal an rAAV particle comprising an AAV capsid protein and a vector comprising a nucleic acid encoding a protective ApoE isoform protein inserted between a pair of AAV inverted terminal repeats, thereby delivering the nucleic acid to the ependymal cell, wherein the ependymal cell secretes the ApoE protein so as to treat the disease. Disclosed is a method of delivering a nucleic acid to an ependymal cell in a mammal comprising administering to the mammal an AAV particle comprising the nucleic acid inserted between a pair of AAV inverted terminal repeats, thereby delivering the nucleic acid to an ependymal cell in the mammal.

In certain embodiments, disclosed is a method of delivering a nucleic acid encoding a protective ApoE isoform to an ependymal cell of a mammal comprising administering to the ependymal cell an rAAV particle comprising an AAV capsid protein and a vector comprising the nucleic acid inserted between a pair of AAV inverted terminal repeats, thereby delivering the nucleic acid to the ependymal cell.

In certain embodiments, disclosed is a method of delivering a nucleic acid encoding a protective ApoE isoform to a mammal comprising administering to an ependymal cell from the mammal an rAAV particle comprising an AAV capsid protein and a vector comprising the nucleic acid inserted between a pair of AAV inverted terminal repeats, and returning the ependymal cell to the mammal, thereby delivering the nucleic acid to the mammal.

In certain embodiments, disclosed is a method of delivering a nucleic acid encoding a protective ApoE isoform to an ependymal cell in a mammal comprising administering to the mammal an rAAV particle comprising an AAV capsid protein and a vector comprising the nucleic acid inserted between a pair of AAV inverted terminal repeats, thereby delivering the nucleic acid to an ependymal cell in the mammal.

In certain embodiments, disclosed is a method of transfecting an ependymal cell a mammalian brain comprising administering to the cerebrospinal fluid (CSF) of the mammal an rAAV particle comprising an AAV capsid protein and a vector comprising a nucleic acid encoding a protective ApoE isoform inserted between a pair of AAV inverted terminal repeats in a manner effective to infect ependymal cells in the mammal such that the ependymal cells secrete the agent into the CSF of the mammal.

In certain embodiments, the mammal is a non-rodent mammal. In certain embodiments, the non-rodent mammal is a primate, horse, sheep, goat, pig, or dog. In certain embodiments, the primate is a human.

In certain embodiments, the protective ApoE isoform has at least about 80% homology to ApoE ε2. In certain embodiments, the protective ApoE isoform has 100% homology to ApoE ε2.

In certain embodiments, the AAV particle is an rAAV4 particle. In certain embodiments, the AAV particle is an rAAV2 particle. In certain embodiments, the rAAV2 capsid has at least 80% homology to AAV2 capsid protein VP1, VP2, and/or VP3. In certain embodiments, the rAAV2 capsid has 100% homology to AAV2 capsid VP1, VP2, and/or VP3.

In certain embodiments, disclosed is an rAAV particle containing a vector comprising a nucleic acid encoding a protective ApoE isoform inserted between a pair of AAV inverted terminal repeats for use in the transfection of ependymal cells in a mammal to generate a therapeutic result.

In certain embodiments, disclosed is a use of an rAAV particle containing a vector comprising a nucleic acid encoding a protective ApoE isoform inserted between a pair of AAV inverted terminal repeats for the manufacture of a medicament useful for the treatment of or prevention of Alzheimer's disease in an animal, such as a human.

Disclosed is a cell as described hereinabove for use in medical treatment or diagnosis.

Disclosed is a use of the cell as described hereinabove to prepare a medicament useful for treating Alzheimer's disease in a mammal.

In certain embodiments, disclosed is a kit comprising a compound of rAAV particle containing a vector comprising a nucleic acid encoding a protective ApoE isoform inserted between a pair of AAV inverted terminal repeats, a container, and a package insert or label indicating the administration of the AAV particle to the CSF for treating Alzheimer's disease in an animal.

### BRIEF DESCRIPTION OF THE DRAWINGS

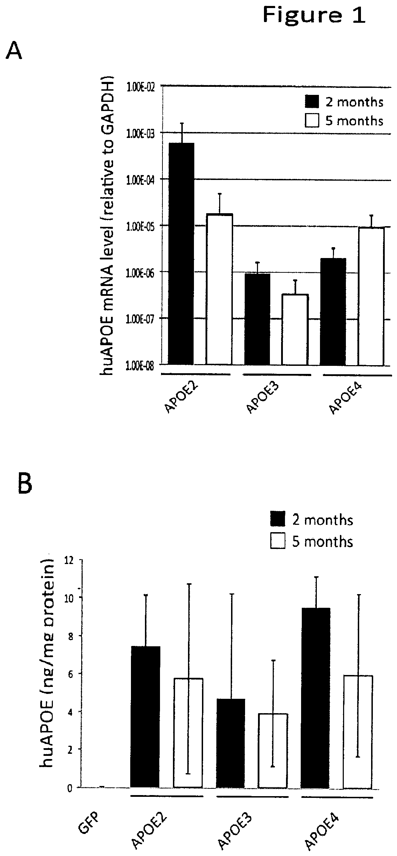

**Figures 1A****-1B.** Intraventricular injection of AAV4-ApoE leads to a stable expression of huAPOE and a sustained detection of recombinant huApoE protein in the brain.

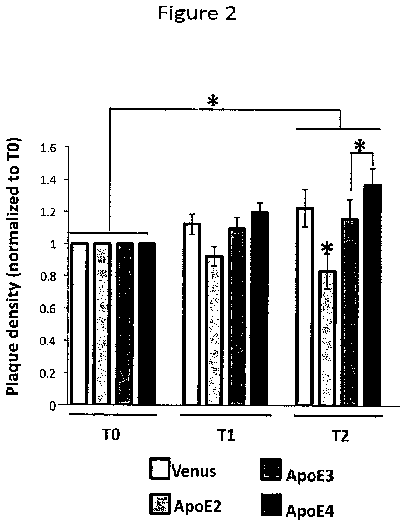

**Figure 2**. Overexpression of each isoform of ApoE differentially affects the progression of the amyloidosis.

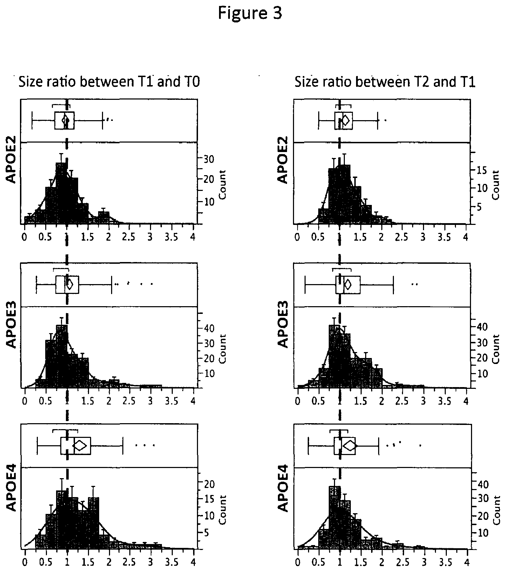

**Figure 3****.** The sizes of amyloid plaques vary according to each ApoE isoform.

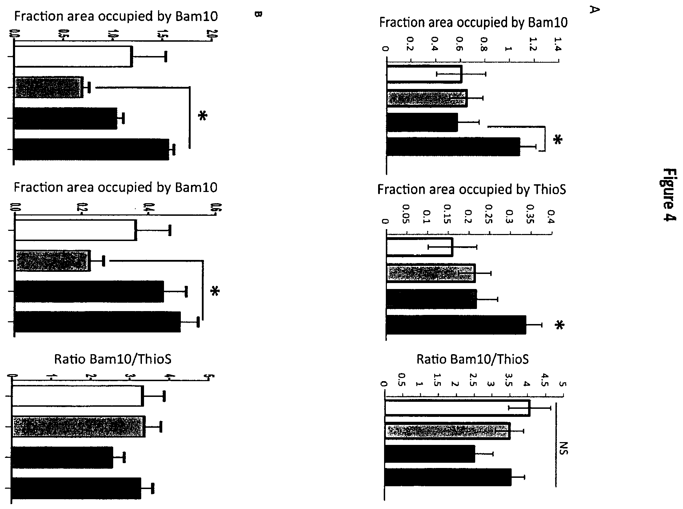

**Figures 4A****-4B.** Post-mortem evaluation of amyloid load confirms the effects of ApoE2 and ApoE4 on amyloid deposition.

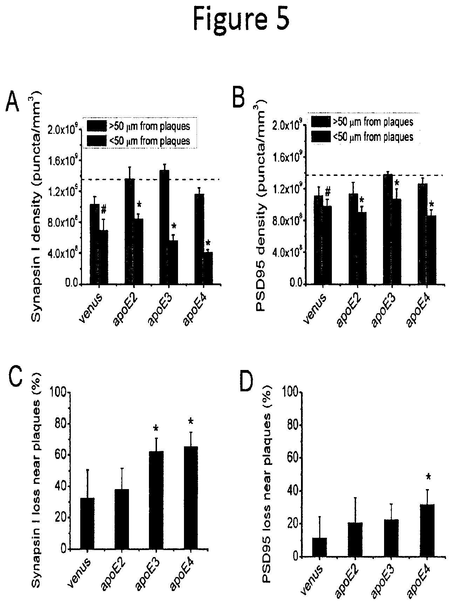

**Figure 5A****-5D.** Each ApoE isform differentially affects synaptic density around amyloid deposits.

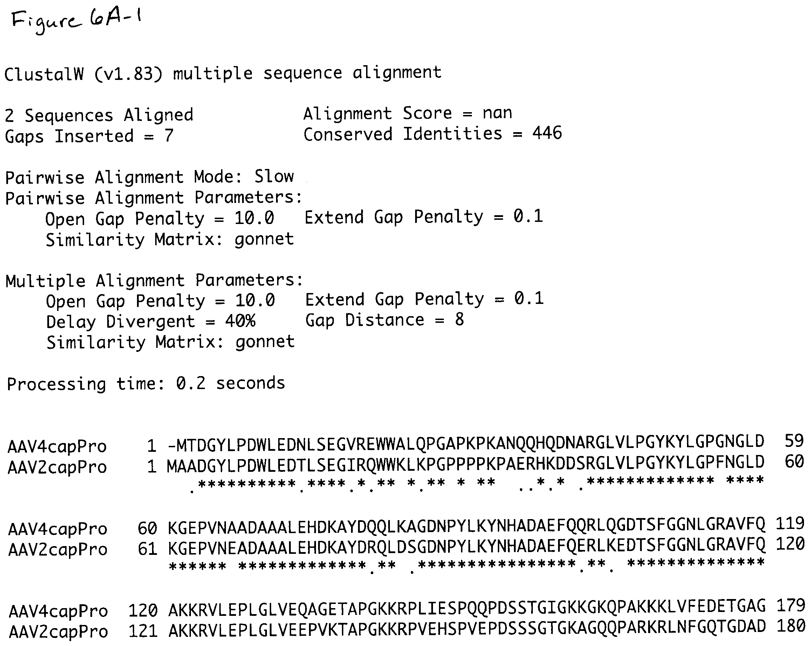

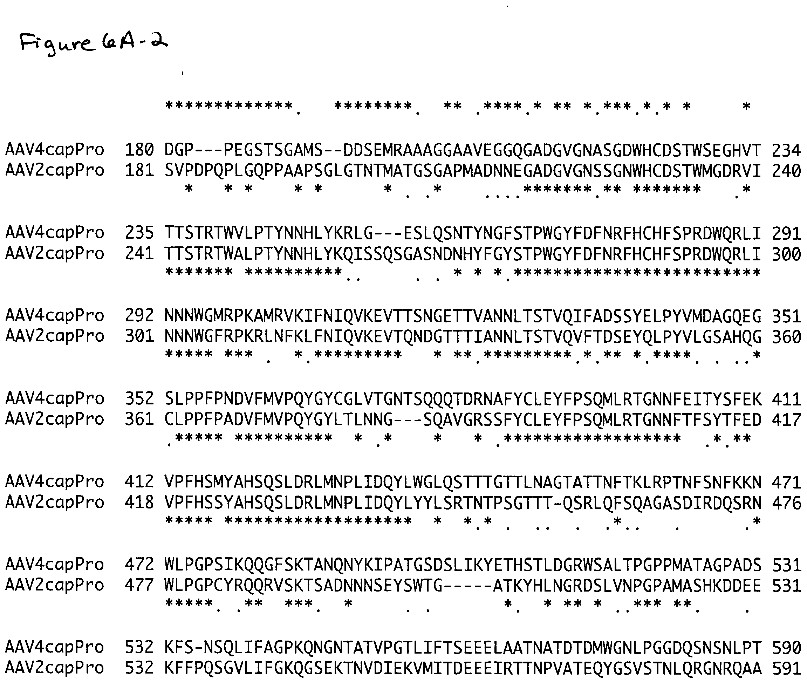

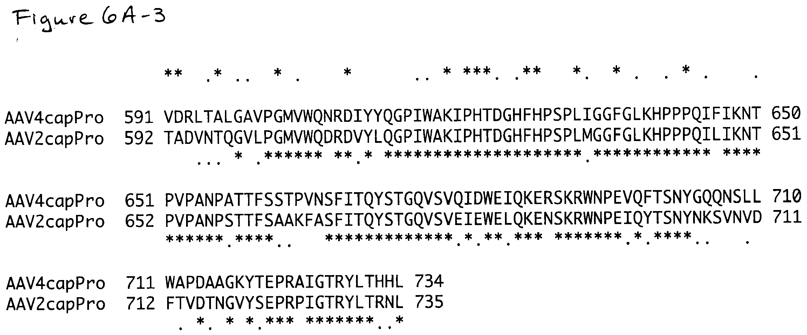

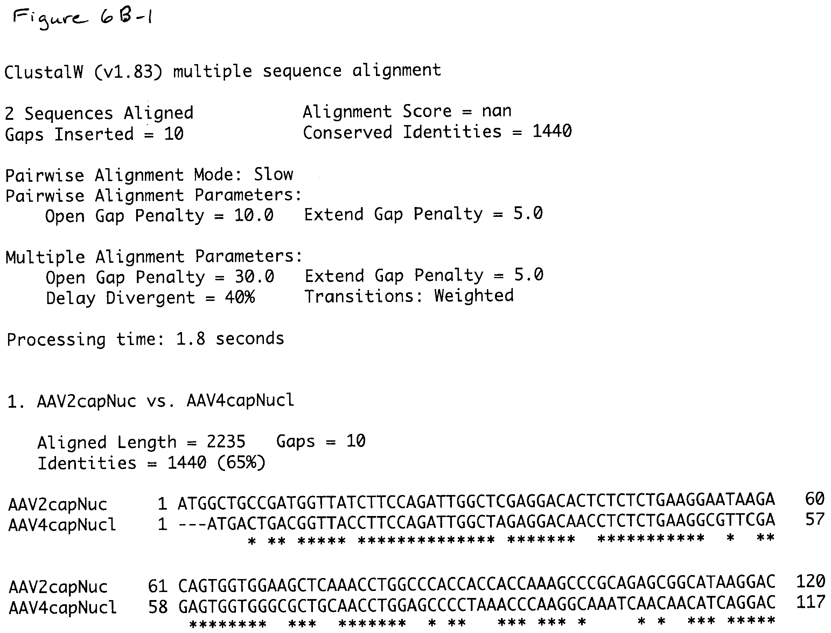

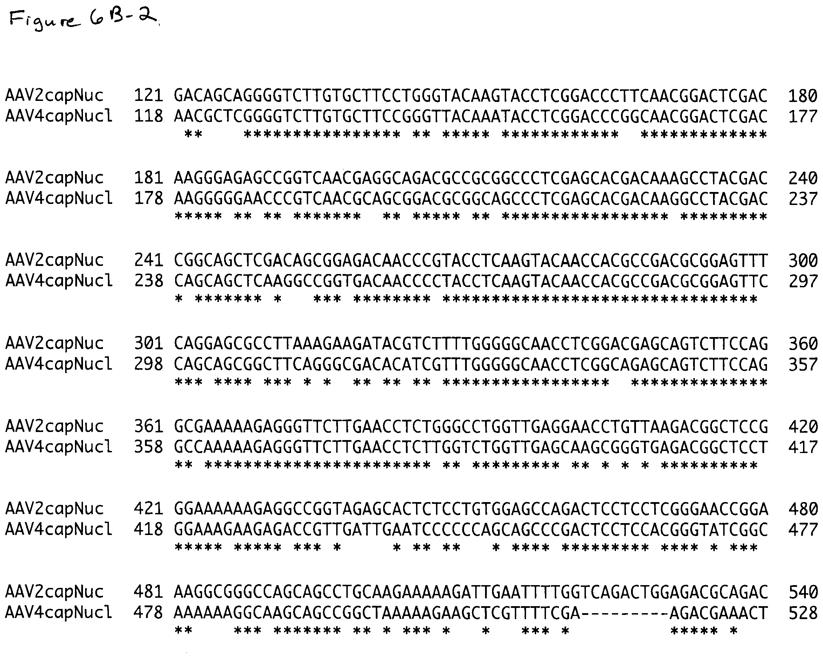

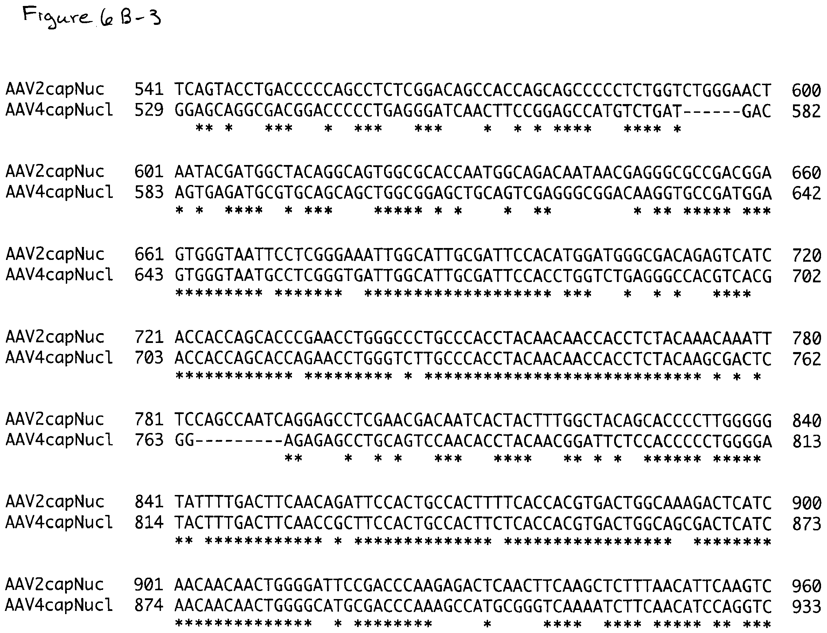

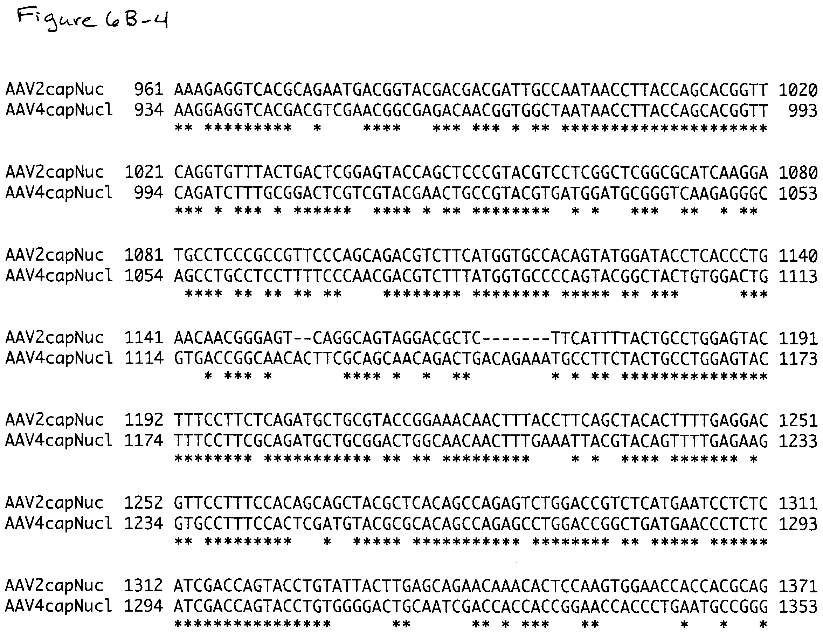

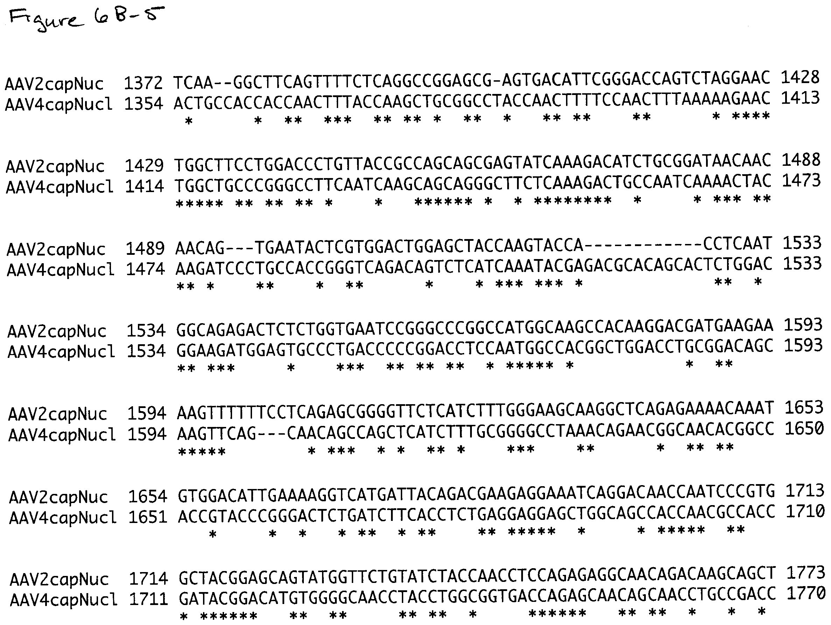

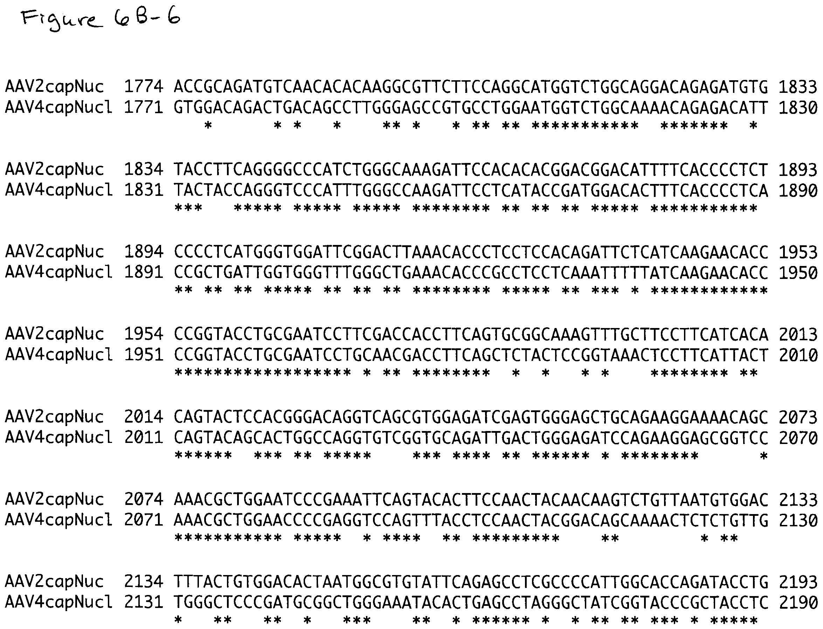

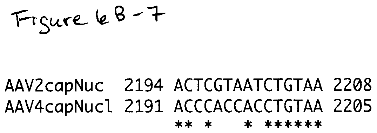

**Figure 6A** is an alignment of AAV2 (SEQ ID NO:1) and AAV4 (SEQ ID NO:2) proteins and **Figure 6B** is and alignment of AAV2 (SEQ ID NO:3) and AAV4 (SEQ ID NO:4) nucleotides based on the sequence from AAV2 (NC_001401) and AAV4 (NC_001829).

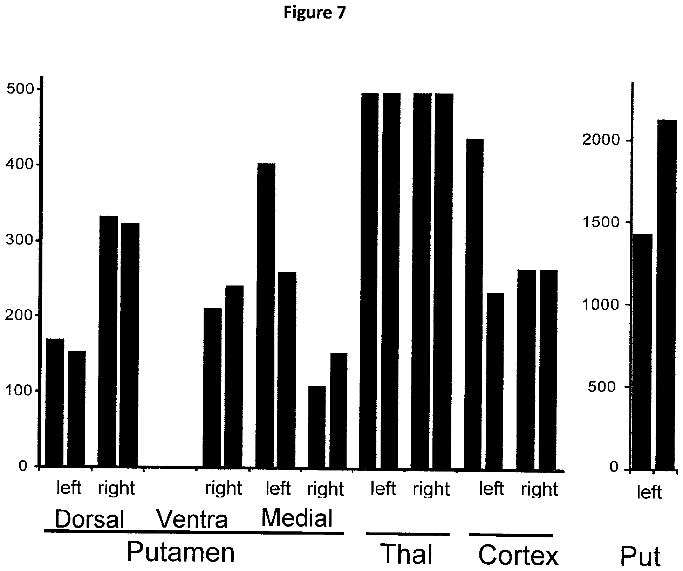

**Figure 7** shows elevated TPP1 activity in various brain regions.

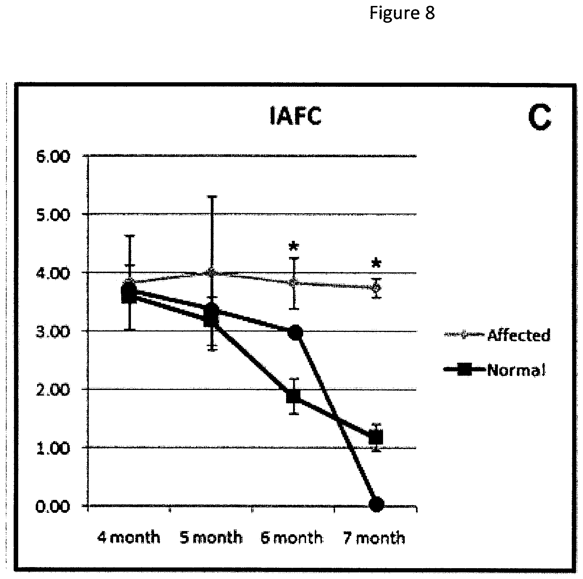

**Figure 8** shows the results of T-maze performance of control and treated dogs. Light circles are for affected dogs; dark squares are for normal dogs, and dark circles are for a TPP-/- dog treated with AAV2-CLN2.

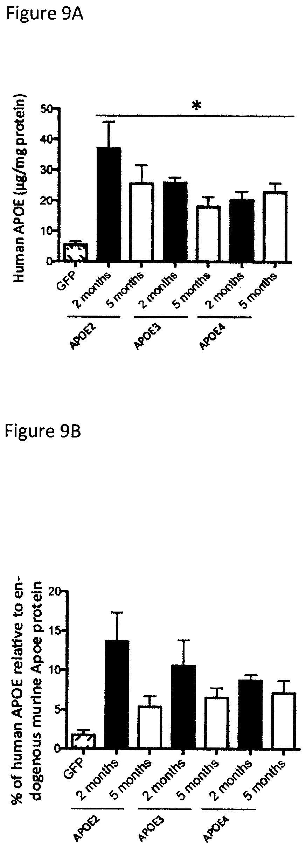

**Figures 9A****-9B.** **Figure 9A****-9B. Validation of the *APOE* gene transfer approach by intraventricular injection of an AAV serotype 4.** Immunohistological labeling of GFP or ApoE revealed the presence of GFP or the human ApoE protein in the ependyma and in the choroid plexus. (A) Use of a species-specific ELISA assay to quantify the concentrations of recombinant human ApoE protein within the cerebral homogenates of injected mice. (B) Evaluation of the percentage of human ApoE protein compared with endogenous apoE per mouse. The ratio of human ApoE and murine endogenous apoE was calculated for each animal. Using the specific anti-human ApoE antibody 3H1, the presence of recombinant protein could be detected around some amyloid deposits where it tends to accumulate, within the cortical parenchyma of APP/PS1 injected mice. Detection of ApoE by Western Blot in the ISF sample of *apoE* KO mice injected with an AAV4-APOE4 vector. The highly sensitive (but non-species specific) Goat anti-apoE antibody from Millipore (AB947) was used as a detection antibody. Albumin was used as a control. n= 4-6 animals per group. *p<0.05.

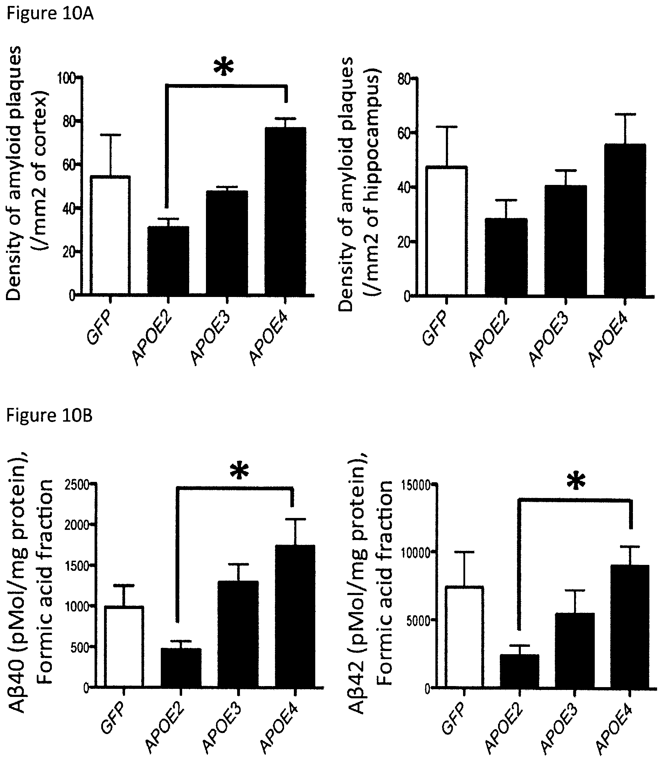

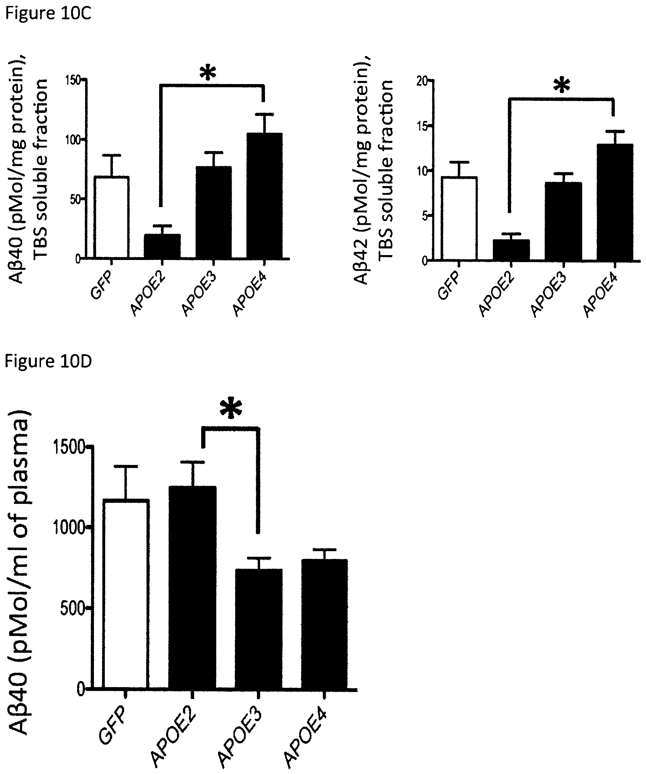

**Figures 10A****-10D. The levels of Aβ peptides and the density of amyloid deposits are modulated by the overexpression of different *APOE* alleles.** (A) Analysis of the density of amyloid deposits in the cortex (left panel) and hippocampus (right panel) of injected transgenic mice. A similar trend could be observed between both cerebral areas, but the data only reached statistical significance in the cortex. (B) Determination by ELISA of the concentrations of Aβ₄₀ and Aβ₄₂ peptides in the formic acid (FA) fraction. (C) Quantification by ELISA of the levels of Aβ₄₀ and Aβ₄₂ peptides in the TBS soluble fraction 5 months after intraventricular injection of each AAV. (D) Quantification of the plasma levels of Aβ₄₀ peptides, 5 months after intraventricular injection of APP/PS 1 mice with AAV-GFP and AAV-APOE2/3/4 vectors. n= 4-7 animals per group. *p<0.05

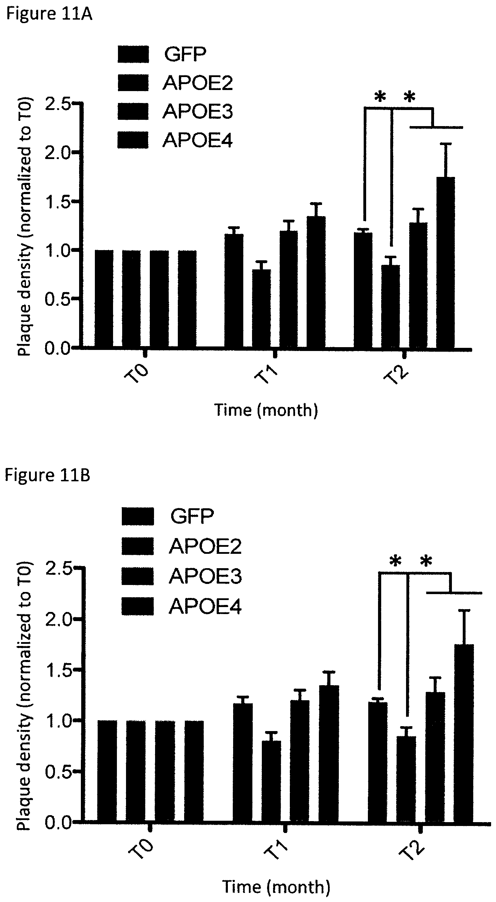

**Figures 11A****-11B. Overexpression of each *APOE* variant differentially modulates the progression of amyloidosis** ***in vivo.** In vivo* two-photon images were developed of amyloid deposition in APP/PS 1 mice one week (T0), one month (T1) and two months (T2) after intracerebroventricular injection with AAV-GFP, -APOE2, -3 or -4 vectors. An intravenous injection of Dextran, Texas red (70,000 Da) was performed prior imaging so that the same fields of view could be followed over time. Within a two month period of time, few new amyloid plaques could be detected, whereas occasional deposits initially visible were not detectable anymore after one or two months. (A) Evaluation of the volumetric cortical density of amyloid deposits over a two-month period of time after intraventricular injection of an AAV-GFP, -APOE2, -APOE3 or APOE4 in 7 month-old APP/PS1 mice. Six to eight fields of view were longitudinally imaged for each animal and the density of plaques was calculated per volume of cortex and reported to the initial value for each animal at baseline (T0). An overall progression of 0.23 of the density of amyloid deposits was observed over time (T2/T1, p<0.011). In addition, ApoE2 significantly reduces the density relative to GFP by 0.66 (se=0.21, p=0.002), relative to ApoE3 by 0.67 (se=0.17, p<0.0001) and relative to ApoE4 by 0.74 (se=0.17, p<0.0001). (B) Linear regression fit of amyloidosis progression over 2 months after gene transfer in APP/PS 1 mice shows that only AAV-APOE4 induces a significant positive slope during this period of time. n= 4-6 animals per group. *p<0.05.

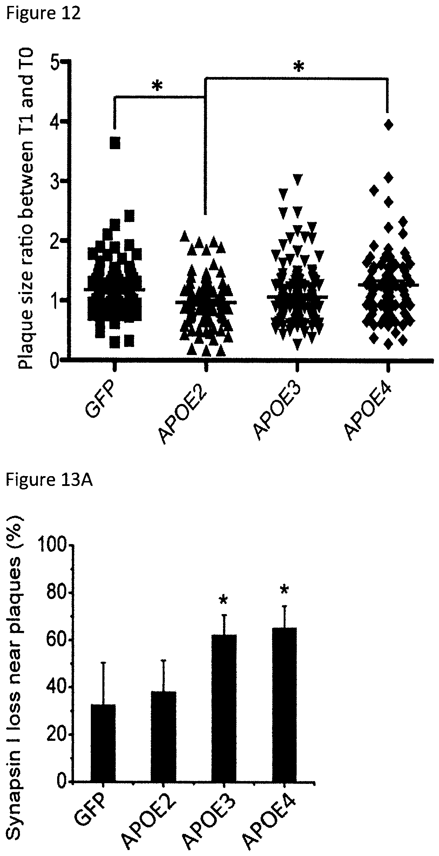

**Figure 12****. Evolution of the size of amyloid deposits one and two month(s) after infusion with ApoE2, -3 and -4.** Scatter dot plots representing the ratio of plaque sizes between T1 and T0 showed that ApoE4 was associated with increased plaque growth compared with both ApoE2 and ApoE3 after one month. This effect is not sustained after 2 months. n> 50 plaques measured per group within 3 to 4 animals. *p<0.05.

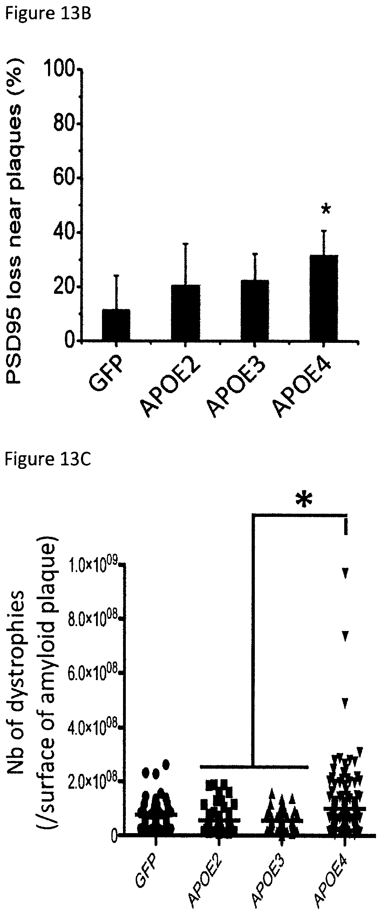

**Figures 13A****-13C. The neuropathological changes associated with the amyloid deposits are differentially affected by each APOE variant.** Images of array tomography sections immunostained for PSD95 (post-synaptic element) and amyloid deposits in APP/PS1 mice 2 months after intraventricular injection of AAV-GFP, -APOE2, -APOE3 and -APOE4 were prepared. Amyloid deposits were labeled using the antibody NAB61 that was previously shown to preferentially label toxic Aβ oligomeric species (A) A significantly higher loss of the synapsin-1 marker was observed in the vicinity of amyloid plaques when both *APOE3* and *APOE4* were expressed compared with *GFP* or *APOE2.* (B) A similar effect was observed when post-synaptic elements were quantified, so that the density of PSD95 surrounding the deposits was decreased 2 months after an intraventricular injection of AAV4-APOE4. As an additional parameter of neuropathological change, the number of neuritic dystrophies per amyloid plaque was evaluated in the brain of injected APP/PS 1 mice, after immunostaining for ThioS and the axonal marker SMI312. (C) A significant shift toward a higher number of dystrophies was observed when mice were infused with ApoE4 was expressed compared with ApoE3 and ApoE2 groups, thus suggesting that ApoE4 may have deleterious effects beyond amyloid plaques formation and may modulate the neurotoxic potential of smaller oligomeric amyloid aggregates. n= 4-6 animals per group. *p<0.05.

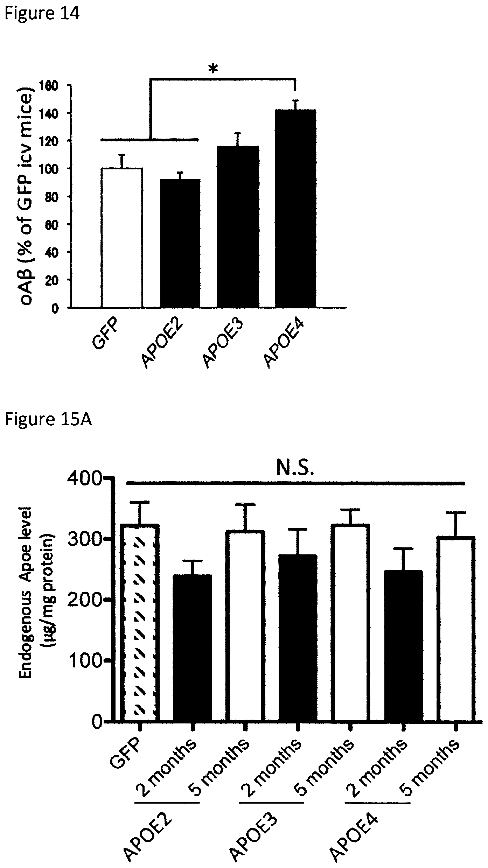

**Figure 14****. Early changes in the content of oligomeric Aβ species are observed in the ISF after intracerebroventricular injection of AAV4-APOE2, -3, -4 in Tg2576 mice.** Quantification of the ISF content in οAβ using the 82E1/82E1 ELISA assay shows that there is a higher concentration of oligomeric amyloid β species after injection of AAV4-APOE4 compared with AAV4-APOE2 and -GFP, whereas AAV4-APOE3 injected mice reached an intermediate level. n= 3-6 animals per group. *p<0.05.
**Figure 15A****-15B. Detection of human and endogenous murine APOE mRNA and protein after intraventricular injection of an AAV4 in APP/PS1 mice.** (A) Box blot graphs representing the amounts of endogenous murine apoE protein in the brains of injected mice. (B) Comparison of the levels of ApoE protein 2 and 5 months after intracerebroventricular injection of AAV4 in APP/PS1 mice (samples from all ApoE injected mice were pooled together at 2 and 5 months, without discrimination for the APOE variant). n= 4-6 animals per group. *p<0.05.

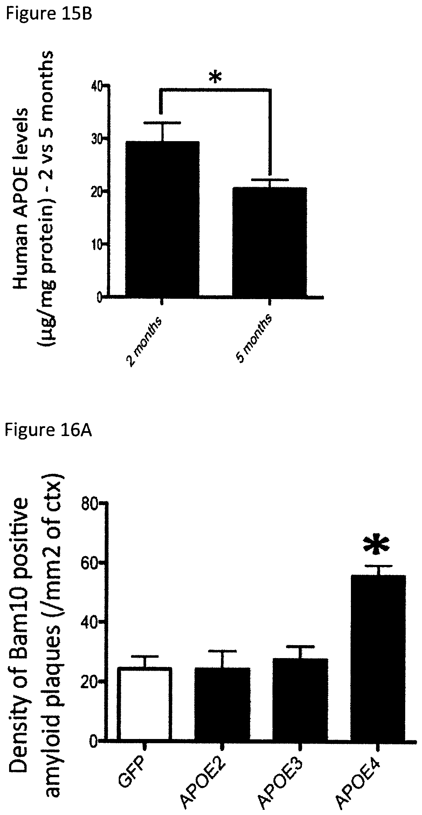

**Fig. 16A****-16B. Effects on Aβ are associated with each ApoE isoform after a short (2 month) exposure.** Images were prepared of amyloid deposition in APP/PS 1 mice 2 months after injection. Both immunostaining using the Bam10 antibody and ThioS were used to stain all amyloid deposits or dense-core plaques respectively. (A) Stereological analysis of the density of amyloid deposits in the cortex revealed that overexpression of *APOE4* led to an increased number of plaques as early as 2 months after injection, whereas no difference could be observed between the other experimental groups. (B) The ratio between Bam10 and ThioS staining, on the other hand, remain unchanged among all the different groups. (C) Determination of the concentrations of Aβ₄₀ (left panels) and Aβ₄₂ (right panels) peptides in the insoluble formic acid extracts after a short exposure with the different ApoE variants. n= 3-5 animals per group. *p<0.05.

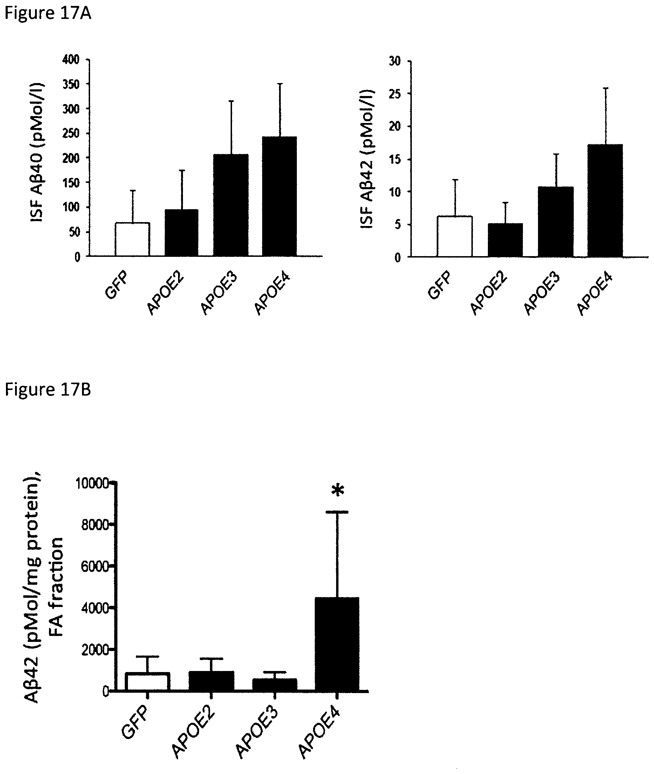

**Figures 17A****-17B. Changes in soluble and insoluble Aβ species detected 3 months after injection in Tg2576 mice.** (A) Quantification by ELISA of the ISF content in Aβ₄₀ and Aβ₄₂ (B) shows that there is a tendency towards higher concentration of soluble amyloid β peptides after injection of AAV4-APOE4 compared with AAV4-APOE2, -APOE3 and -GFP. (B) As previously observed in APP/PS1 mice, the stronger effect was observed with ApoE4, which causes significantly higher amounts of Aβ₄₂ in the formic acid fraction of Tg2576 mice. n= 3-5 animals per group. *p<0.05.

### DETAILED DESCRIPTION

The present invention relates to an rAAV2 particle comprising an AAV2 capsid protein and a vector comprising a nucleic acid encoding a protective ApoE ε2 isoform protein inserted between a pair of AAV2 inverted terminal repeats, for use in treating Alzheimer's disease in a mammal, wherein the rAAV particle is administered to an ependymal cell thereby delivering the nucleic acid to the ependymal cell, wherein the ependymal cell secretes the ApoE ε2 protein so as to treat the disease, and wherein the mammal is a non-rodent mammal.

The present invention further relates to a pharmaceutical composition comprising an rAAV2 particle comprising an AAV2 capsid protein and a vector comprising a nucleic acid encoding a protective ApoE ε2 isoform protein inserted between a pair of AAV2 inverted terminal repeats, for use in treating Alzheimer's disease in a mammal, wherein the rAAV2 particle is administered to an ependymal cell thereby delivering the nucleic acid to the ependymal cell, wherein the ependymal cell secretes the ApoE ε2 protein so as to treat the disease, and wherein the mammal is a non-rodent mammal.

There are several different human apolipoprotein E (ApoE) isoforms, the presence of some of these isoforms in the brain increase the risk for Alzheimer's disease (AD), whereas the presence of other isoforms decreases the risk for AD. The presence of the ApoE ε4 isoform is a strong genetic risk factor for late-onset, sporadic AD. (Casellano et al., Sci Transl Med, 3(89):89ra57 (29 June 2011).) The ApoE ε4 allele strongly increases AD risk and decreases age of onset. On the other hand, the presence of the ApoE ε2 allele appears to decrease AD risk. It is suggested that human ApoE isoforms differentially affect the clearance or synthesis of amyloid-β (Aβ) *in vivo.*

Adeno associated virus (AAV) is a small nonpathogenic virus of the parvoviridae family. AAV is distinct from the other members of this family by its dependence upon a helper virus for replication. In the absence of a helper virus, AAV may integrate in a locus specific manner into the q arm of chromosome 19. The approximately 5 kb genome of AAV consists of one segment of single stranded DNA of either plus or minus polarity. The ends of the genome are short inverted terminal repeats which can fold into hairpin structures and serve as the origin of viral DNA replication. Physically, the parvovirus virion is non-enveloped and its icosohedral capsid is approximately 20 nm in diameter.

To-date eight serologically distinct AAVs have been identified and five have been isolated from humans or primates and are referred to as AAV types 1-5. Govindasamy et al., "Structurally Mapping the Diverse Phenotype of Adeno-Associated Virus Serotype 4," J. Vir., 80 (23):11556-11570 (2006). The genome of AAV2 is 4680 nucleotides in length and contains two open reading frames (ORFs). The left ORF encodes the non-structural Rep proteins, Rep 40, Rep 52, Rep 68 and Rep 78, which are involved in regulation of replication and transcription in addition to the production of single-stranded progeny genomes. Furthermore, two of the Rep proteins have been associated with the preferential integration of AAV genomes into a region of the q arm of human chromosome 19. Rep68/78 has also been shown to possess NTP binding activity as well as DNA and RNA helicase activities. The Rep proteins possess a nuclear localization signal as well as several potential phosphorylation sites. Mutation of one of these kinase sites resulted in a loss of replication activity.

The ends of the genome are short inverted terminal repeats (ITR) which have the potential to fold into T-shaped hairpin structures that serve as the origin of viral DNA replication. Within the ITR region two elements have been described which are central to the function of the ITR, a GAGC repeat motif and the terminal resolution site (trs). The repeat motif has been shown to bind Rep when the ITR is in either a linear or hairpin conformation. This binding serves to position Rep68/78 for cleavage at the trs which occurs in a site- and strand-specific manner. In addition to their role in replication, these two elements appear to be central to viral integration. Contained within the chromosome 19 integration locus is a Rep binding site with an adjacent trs. These elements have been shown to be functional and necessary for locus specific integration.

The AAV2 virion is a non-enveloped, icosohedral particle approximately 25 nm in diameter, consisting of three related proteins referred to as VP1, VP2 and VP3. The right ORF encodes the capsid proteins VP1, VP2, and VP3. These proteins are found in a ratio of 1:1:10 respectively and are all derived from the right-hand ORF. The capsid proteins differ from each other by the use of alternative splicing and an unusual start codon. Deletion analysis has shown that removal or alteration of VP1 which is translated from an alternatively spliced message results in a reduced yield of infections particles. Mutations within the VP3 coding region result in the failure to produce any single-stranded progeny DNA or infectious particles. An AAV2 particle is a viral particle comprising an AAV2 capsid protein. An AAV2 capsid polypeptide can encode the entire VP1, VP2 and VP3 polypeptide. The particle can be a particle comprising AAV2 and other AAV capsid proteins (i.e., a chimeric protein, such as AAV4 and AAV2). Variations in the amino acid sequence of the AAV2 capsid protein are contemplated herein, as long as the resulting viral particle comprises the AAV2 capsid remains antigenically or immunologically distinct from AAV4, as can be routinely determined by standard methods. Specifically, for example, ELISA and Western blots can be used to determine whether a viral particle is antigenically or immunologically distinct from AAV4. Furthermore, the AAV2 viral particle preferably retains tissue tropism distinct from AAV4.

An AAV2 particle is a viral particle comprising an AAV2 capsid protein. An AAV2 capsid polypeptide encoding the entire VP1, VP2, and VP3 polypeptide can overall have at least about 63% homology (or identity) to the polypeptide having the amino acid sequence encoded by nucleotides set forth in SEQ ID NO:1 (AAV2 capsid protein). The capsid protein can have about 70% homology, about 75% homology, 80% homology, 85% homology, 90% homology, 95% homology, 98% homology, 99% homology, or even 100% homology to the protein set forth in SEQ ID NO:1. The capsid protein can have about 70% identity, about 75% identity, 80% identity, 85% identity, 90% identity, 95% identity, 98% identity, 99% identity, or even 100% identity to the protein set forth in SEQ ID NO:1. The particle can be a particle comprising both AAV4 and AAV2 capsid protein, i.e., a chimeric protein. Variations in the amino acid sequence of the AAV2 capsid protein are contemplated herein, as long as the resulting viral particle comprising the AAV2 capsid remains antigenically or immunologically distinct from AAV4, as can be routinely determined by standard methods. Specifically, for example, ELISA and Western blots can be used to determine whether a viral particle is antigenically or immunologically distinct from AAV4. Furthermore, the AAV2 viral particle preferably retains tissue tropism distinction from AAV4, such as that exemplified in the examples herein, though an AAV2 chimeric particle comprising at least one AAV2 coat protein may have a different tissue tropism from that of an AAV2 particle consisting only of AAV2 coat proteins.

As indicated in **Figures 6A** **and** **6B**, AAV2 capsid sequence and AAV4 capsid sequence are about 60% homologous. In certain embodiments, the AAV2 capsid comprises (or consists of) a sequence that is at least 65% homologous to the amino acid sequence set forth in SEQ ID NO:1.

In certain embodiments, disclosed is an AAV2 particle containing, i.e., encapsidating, a vector comprising a pair of AAV2 inverted terminal repeats. The nucleotide sequence of AAV2 ITRs is known in the art. Furthermore, the particle can be a particle comprising both AAV4 and AAV2 capsid protein, i.e., a chimeric protein. Moreover, the particle can be a particle encapsidating a vector comprising a pair of AAV inverted terminal repeats from other AAVs (e.g., AAV1-AAV8). The vector encapsidated in the particle can further comprise an exogenous nucleic acid inserted between the inverted terminal repeats.

The following features of AAV have made it an attractive vector for gene transfer. AAV vectors have been shown in vitro to stably integrate into the cellular genome; possess a broad host range; transduce both dividing and non dividing cells in vitro and in vivo and maintain high levels of expression of the transduced genes. Viral particles are heat stable, resistant to solvents, detergents, changes in pH, temperature, and can be concentrated on CsCl gradients. Integration of AAV provirus is not associated with any long term negative effects on cell growth or differentiation. The ITRs have been shown to be the only cis elements required for replication, packaging and integration and may contain some promoter activities.

Disclosed are methods of administering AAV particles, recombinant AAV vectors, and recombinant AAV virions. For example, an AAV2 particle is a viral particle comprising an AAV2 capsid protein, or an AAV4 particle is a viral particle comprising an AAV4 capsid protein. A recombinant AAV2 vector is a nucleic acid construct that comprises at least one unique nucleic acid of AAV2. A recombinant AAV2 virion is a particle containing a recombinant AAV2 vector. To be considered within the term "AAV2 ITRs" the nucleotide sequence must retain one or both features described herein that distinguish the AAV2 ITR from the AAV4 ITR: (1) three (rather than four as in AAV4) "GAGC" repeats and (2) in the AAV2 ITR Rep binding site the fourth nucleotide in the first two "GAGC" repeats is a C rather than a T.

The promoter can be any desired promoter, selected by known considerations, such as the level of expression of a nucleic acid functionally linked to the promoter and the cell type in which the vector is to be used. Promoters can be an exogenous or an endogenous promoter. Promoters can include, for example, known strong promoters such as SV40 or the inducible metallothionein promoter, or an AAV promoter, such as an AAV p5 promoter. Additional examples of promoters include promoters derived from actin genes, immunoglobulin genes, cytomegalovirus (CMV), adenovirus, bovine papilloma virus, adenoviral promoters, such as the adenoviral major late promoter, an inducible heat shock promoter, respiratory syncytial virus, Rous sarcomas virus (RSV), etc. Specifically, the promoter can be AAV2 p5 promoter or AAV4 p5 promoter. Furthermore, smaller fragments of p5 promoter that retain promoter activity can readily be determined by standard procedures including, for example, constructing a series of deletions in the p5 promoter, linking the deletion to a reporter gene, and determining whether the reporter gene is expressed, i.e., transcribed and/or translated.

The AAV vector can further comprise an exogenous (heterologous) nucleic acid functionally linked to the promoter. By "heterologous nucleic acid" is meant that any heterologous or exogenous nucleic acid can be inserted into the vector for transfer into a cell, tissue or organism. For example, in certain embodiments, the heterologous nucleic acid encodes a protective ApoE isoform. By "functionally linked" is meant such that the promoter can promote expression of the heterologous nucleic acid, as is known in the art, such as appropriate orientation of the promoter relative to the heterologous nucleic acid. Furthermore, the heterologous nucleic acid preferably has all appropriate sequences for expression of the nucleic acid, as known in the art, to functionally encode, i.e., allow the nucleic acid to be expressed. The nucleic acid can include, for example, expression control sequences, such as an enhancer, and necessary information processing sites, such as ribosome binding sites, RNA splice sites, polyadenylation sites, and transcriptional terminator sequences. The nucleic acid can encode more than one gene product, limited only by the size of nucleic acid that can be packaged.

An AAV2 particle is a viral particle comprising an AAV2 capsid protein. Variations in the amino acid sequence of the AAV2 capsid protein are contemplated herein, as long as the resulting viral particle comprising the AAV2 capsid remains antigenically or immunologically distinct from AAV4, as can be routinely determined by standard methods. Specifically, for example, ELISA and Western blots can be used to determine whether a viral particle is antigenically or immunologically distinct from other AAV serotypes.

AAV4 is a unique member of the AAV family. A discussion of AAV4 is provided in US Patent No. 6,468,524. DNA hybridization data indicated a similar level of homology for AAV1-4. However, in contrast to the other AAVs, only one ORF corresponding to the capsid proteins was identified in AAV4 and no ORF was detected for the Rep proteins. Disclosed is a vector comprising the AAV4 virus as well as AAV4 viral particles. While AAV4 is similar to AAV2, the two viruses are found herein to be physically and genetically distinct. These differences endow AAV4 with some unique advantages which better suit it as a vector for gene therapy. For example, the wildtype AAV4 genome is larger than AAV2, allowing for efficient encapsidation of a larger recombinant genome. Furthermore, wildtype AAV4 particles have a greater buoyant density than AAV2 particles and therefore are more easily separated from contaminating helper virus and empty AAV particles than AAV2-based particles. Additionally, in contrast to AAV1, 2, and 3, AAV4 is able to hemagglutinate human, guinea pig, and sheep erythrocytes.

In certain embodiments, disclosed is a vector comprising the AAV5 virus or a vector comprising subparts of the virus, as well as AAV5 viral particles. A discussion of AAV5 is provided in US Patent No. 6,855,314. While AAV5 is similar to AAV2, the two viruses are found herein to be physically and genetically distinct. These differences endow AAV5 with some unique properties and advantages which better suit it as a vector for gene therapy. For example, one of the limiting features of using AAV2 as a vector for gene therapy is production of large amounts of virus. Using standard production techniques, AAV5 is produced at a 10-50 fold higher level compared to AAV2. Because of its unique TRS site and rep proteins, AAV5 should also have a distinct integration locus compared to AAV2.

Furthermore, AAV5 capsid protein, again surprisingly, is distinct from AAV2 capsid protein and exhibits different tissue tropism, thus making AAV5 capsid-containing particles suitable for transducing cell types for which AAV2 is unsuited or less well-suited. AAV2 and AAV5 have been shown to be serologically distinct and thus, in a gene therapy application, AAV5, and AAV5-derived vectors, would allow for transduction of a patient who already possess neutralizing antibodies to AAV2 either as a result of natural immunological defense or from prior exposure to AAV2 vectors. Another advantage of AAV5 is that AAV5 cannot be rescued by other serotypes. Only AAV5 can rescue the integrated AAV5 genome and effect replication, thus avoiding unintended replication of AAV5 caused by other AAV serotypes.

The term "polypeptide" as used herein refers to a polymer of amino acids and includes full-length proteins and fragments thereof. Thus, "protein," polypeptide," and "peptide" are often used interchangeably herein. Substitutions can be selected by known parameters to be neutral. As will be appreciated by those skilled in the art, the disclosure also includes those polypeptides having slight variations in amino acid sequences or other properties. Such variations may arise naturally as allelic variations (e.g. due to genetic polymorphism) or may be produced by human intervention (e.g., by mutagenesis of cloned DNA sequences), such as induced point, deletion, insertion and substitution mutants. Minor changes in amino acid sequence are generally preferred, such as conservative amino acid replacements, small internal deletions or insertions, and additions or deletions at the ends of the molecules. These modifications can result in changes in the amino acid sequence, provide silent mutations, modify a restriction site, or provide other specific mutations.

The present method provides a method of delivering a nucleic acid to a cell comprising administering to the cell an AAV particle containing a vector comprising the nucleic acid inserted between a pair of AAV inverted terminal repeats, thereby delivering the nucleic acid to the cell. Administration to the cell can be accomplished by any means, including simply contacting the particle, optionally contained in a desired liquid such as tissue culture medium, or a buffered saline solution, with the cells. The particle can be allowed to remain in contact with the cells for any desired length of time, and typically the particle is administered and allowed to remain indefinitely. For such in vitro methods, the virus can be administered to the cell by standard viral transduction methods, as known in the art and as exemplified herein. Titers of virus to administer can vary, particularly depending upon the cell type, but will be typical of that used for AAV transduction in general. Additionally the titers used to transduce the particular cells in the present examples can be utilized. The cells can include any desired cell in humans as well as other large (non-rodent) mammals, such as primates, horse, sheep, goat, pig, and dog.

More specifically, disclosed is a method of delivering a nucleic acid to an ependymal cell, comprising administering to the ependymal cell an AAV particle containing a vector comprising the nucleic acid inserted between a pair of AAV inverted terminal repeats, thereby delivering the nucleic acid to the ependymal cell.

Also disclosed is a method of delivering a nucleic acid to a subject comprising administering to a cell from the subject an AAV particle comprising the nucleic acid inserted between a pair of AAV inverted terminal repeats, and returning the cell to the subject, thereby delivering the nucleic acid to the subject. In certain embodiments, the AAV ITRs can be AAV2 ITRs. For such an ex vivo administration, cells are isolated from a subject by standard means according to the cell type and placed in appropriate culture medium, again according to cell type. Viral particles are then contacted with the cells as described above, and the virus is allowed to transfect the cells. Cells can then be transplanted back into the subject's body, again by means standard for the cell type and tissue. If desired, prior to transplantation, the cells can be studied for degree of transfection by the virus, by known detection means and as described herein.

Further disclosed is a method of delivering a nucleic acid to a cell in a subject comprising administering to the subject an AAV particle comprising the nucleic acid inserted between a pair of AAV inverted terminal repeats, thereby delivering the nucleic acid to a cell in the subject. Administration can be an ex vivo administration directly to a cell removed from a subject, such as any of the cells listed above, followed by replacement of the cell back into the subject, or administration can be in vivo administration to a cell in the subject. For ex vivo administration, cells are isolated from a subject by standard means according to the cell type and placed in appropriate culture medium, again according to cell type. Viral particles are then contacted with the cells as described above, and the virus is allowed to transfect the cells. Cells can then be transplanted back into the subject's body, again by means standard for the cell type and tissue. If desired, prior to transplantation, the cells can be studied for degree of transfection by the virus, by known detection means and as described herein.

Also disclosed is a method of delivering a nucleic acid to an ependymal cell in a subject comprising administering to the subject an AAV particle comprising the nucleic acid inserted between a pair of AAV inverted terminal repeats, thereby delivering the nucleic acid to an ependymal cell in the subject.

In certain embodiments, the amino acid sequence that targets brain vascular endothelium targets brain vascular endothelium in a subject that has a disease, *e.g.,* Alzheimer's disease.

In certain embodiments, the amino acid sequence that targets brain vascular endothelium targets brain vascular endothelium in a subject that does not have Alzheimer's disease.

In certain embodiments, the viral vector comprises a nucleic acid sequence encoding a therapeutic agent. In certain embodiments, the therapeutic agent is a protective ApoE isoform.

Certain embodiments of the present disclosure provide a cell comprising a viral vector as described herein.

In certain embodiments, the cell is a mammalian cell of a non-rodent mammal. In certain embodiments, the cell is a primate cell. In certain embodiments, the cell is a human cell. In certain embodiments, the cell is a non-human cell. In certain embodiments, the cell is *in vitro.* In certain embodiments, the cell is *in vivo.* In certain embodiments, the cell is an ependymal cell.

Certain embodiments of the present disclosure provide a method of treating a disease in a mammal comprising administering a viral vector or the cell as described herein to the mammal.

In certain embodiments, the mammal is human.

Certain embodiments of the present disclosure provide a method to deliver an agent to the central nervous system of a subject, comprising administering to the CSF with a viral vector described herein so that the transduced ependymal cells express the therapeutic agent and deliver the agent to the central nervous system of the subject. In certain embodiments, the viral vector transduces ependymal cells.

Certain embodiments of the present disclosure provide a viral vector or cell as described herein for use in medical treatments.

Certain embodiments of the present disclosure provide a use of a viral vector or cell as described herein to prepare a medicament useful for treating a disease, e.g., Alzheimer's disease, in a mammal.

The vector may further comprise a protective ApoE isoform protein. As used herein, the term "secreted protein" includes any secreted protein, whether naturally secreted or modified to contain a signal sequence so that it can be secreted.

Nucleic acid is "operably linked" when it is placed into a functional relationship with another nucleic acid sequence. Generally, "operably linked" means that the DNA sequences being linked are contiguous. However, enhancers do not have to be contiguous. Linking is accomplished by ligation at convenient restriction sites. If such sites do not exist, the synthetic oligonucleotide adaptors or linkers are used in accordance with conventional practice. Additionally, multiple copies of the nucleic acid encoding enzymes may be linked together in the expression vector. Such multiple nucleic acids may be separated by linkers.

The present disclosure also provides a mammalian cell containing a vector described herein. The cell may be human, and may be from brain. The cell type may be a stem or progenitor cell population.

The present disclosure provides a method of treating a disease such as a genetic disease or cancer in a mammal by administering a polynucleotide, polypeptide, expression vector, or cell described herein. The genetic disease may be a neurodegenerative disease, such as Alzheimer's disease.

Certain aspects of the disclosure relate to polynucleotides, polypeptides, vectors, and genetically engineered cells (modified *in vivo*), and the use of them. In particular, the disclosure relates to a method for gene or protein therapy that is capable of both systemic delivery of a therapeutically effective dose of the therapeutic agent.

According to one aspect, a cell expression system for expressing a therapeutic agent in a mammalian recipient is provided. The expression system (also referred to herein as a "genetically modified cell") comprises a cell and an expression vector for expressing the therapeutic agent. Expression vectors include, but are not limited to, viruses, plasmids, and other vehicles for delivering heterologous genetic material to cells. Accordingly, the term "expression vector" as used herein refers to a vehicle for delivering heterologous genetic material to a cell. In particular, the expression vector is a recombinant adenoviral, adeno-associated virus, or lentivirus or retrovirus vector.

The expression vector further includes a promoter for controlling transcription of the heterologous gene. The promoter may be an inducible promoter (described below). The expression system is suitable for administration to the mammalian recipient. The expression system may comprise a plurality of non-immortalized genetically modified cells, each cell containing at least one recombinant gene encoding at least one therapeutic agent.

The cell expression system can be formed *in vivo.* According to yet another aspect, a method for treating a mammalian recipient *in vivo* is provided. The method includes introducing an expression vector for expressing a heterologous gene product into a cell of the patient *in situ,* such as via intravenous administration. To form the expression system *in vivo,* an expression vector for expressing the therapeutic agent is introduced *in vivo* into the mammalian recipient i.v., where the vector migrates via the vasculature to the brain.

According to yet another aspect, a method for treating a mammalian recipient *in vivo* is provided. The method includes introducing the target protein into the patient *in vivo.*

The expression vector for expressing the heterologous gene may include an inducible promoter for controlling transcription of the heterologous gene product. Accordingly, delivery of the therapeutic agent *in situ* is controlled by exposing the cell *in situ* to conditions, which induce transcription of the heterologous gene.

The mammalian recipient may have a condition that is amenable to gene replacement therapy. As used herein, "gene replacement therapy" refers to administration to the recipient of exogenous genetic material encoding a therapeutic agent and subsequent expression of the administered genetic material *in situ.* Thus, the phrase "condition amenable to gene replacement therapy" embraces conditions such as genetic diseases *(i.e.,* a disease condition that is attributable to one or more gene defects), acquired pathologies *(i.e.,* a pathological condition which is not attributable to an inborn defect), cancers and prophylactic processes *(i.e.,* prevention of a disease or of an undesired medical condition). Accordingly, as used herein, the term "therapeutic agent" refers to any agent or material, which has a beneficial effect on the mammalian recipient. Thus, "therapeutic agent" embraces both therapeutic and prophylactic molecules having nucleic acid or protein components.

According to one embodiment, the mammalian recipient has a genetic disease and the exogenous genetic material comprises a heterologous gene encoding a therapeutic agent for treating the disease. In yet another embodiment, the mammalian recipient has an acquired pathology and the exogenous genetic material comprises a heterologous gene encoding a therapeutic agent for treating the pathology. According to another embodiment, the patient has a cancer and the exogenous genetic material comprises a heterologous gene encoding an anti-neoplastic agent. In yet another embodiment the patient has an undesired medical condition and the exogenous genetic material comprises a heterologous gene encoding a therapeutic agent for treating the condition.

As used herein, the term "a protective ApoE isoform" includes variants or biologically active or inactive fragments of this polypeptide. A "variant" of one of the polypeptides is a polypeptide that is not completely identical to a native protein. Such variant protein can be obtained by altering the amino acid sequence by insertion, deletion or substitution of one or more amino acid. The amino acid sequence of the protein is modified, for example by substitution, to create a polypeptide having substantially the same or improved qualities as compared to the native polypeptide. The substitution may be a conserved substitution. A "conserved substitution" is a substitution of an amino acid with another amino acid having a similar side chain. A conserved substitution would be a substitution with an amino acid that makes the smallest change possible in the charge of the amino acid or size of the side chain of the amino acid (alternatively, in the size, charge or kind of chemical group within the side chain) such that the overall peptide retains its spacial conformation but has altered biological activity. For example, common conserved changes might be Asp to Glu, Asn or Gln; His to Lys, Arg or Phe; Asn to Gln, Asp or Glu and Ser to Cys, Thr or Gly. Alanine is commonly used to substitute for other amino acids. The 20 essential amino acids can be grouped as follows: alanine, valine, leucine, isoleucine, proline, phenylalanine, tryptophan and methionine having nonpolar side chains; glycine, serine, threonine, cystine, tyrosine, asparagine and glutamine having uncharged polar side chains; aspartate and glutamate having acidic side chains; and lysine, arginine, and histidine having basic side chains.

The amino acid changes are achieved by changing the codons of the corresponding nucleic acid sequence. It is known that such polypeptides can be obtained based on substituting certain amino acids for other amino acids in the polypeptide structure in order to modify or improve biological activity. For example, through substitution of alternative amino acids, small conformational changes may be conferred upon a polypeptide that results in increased activity. Alternatively, amino acid substitutions in certain polypeptides may be used to provide residues, which may then be linked to other molecules to provide peptide-molecule conjugates which, retain sufficient properties of the starting polypeptide to be useful for other purposes.

One can use the hydropathic index of amino acids in conferring interactive biological function on a polypeptide, wherein it is found that certain amino acids may be substituted for other amino acids having similar hydropathic indices and still retain a similar biological activity. Alternatively, substitution of like amino acids may be made on the basis of hydrophilicity, particularly where the biological function desired in the polypeptide to be generated in intended for use in immunological embodiments. The greatest local average hydrophilicity of a "protein", as governed by the hydrophilicity of its adjacent amino acids, correlates with its immunogenicity. Accordingly, it is noted that substitutions can be made based on the hydrophilicity assigned to each amino acid.

In using either the hydrophilicity index or hydropathic index, which assigns values to each amino acid, it is preferred to conduct substitutions of amino acids where these values are ±2, with ± 1 being particularly preferred, and those with in ±0.5 being the most preferred substitutions.

The variant protein has at least 50%, at least about 80%, or even at least about 90% but less than 100%, contiguous amino acid sequence homology or identity to the amino acid sequence of a corresponding native protein.

The amino acid sequence of the variant polypeptide corresponds essentially to the native polypeptide's amino acid sequence. As used herein "correspond essentially to" refers to a polypeptide sequence that will elicit a biological response substantially the same as the response generated by the native protein. Such a response may be at least 60% of the level generated by the native protein, and may even be at least 80% of the level generated by native protein.

A variant may include amino acid residues not present in the corresponding native protein or deletions relative to the corresponding native protein. A variant may also be a truncated "fragment" as compared to the corresponding native protein, *i.e.,* only a portion of a full-length protein. Protein variants also include peptides having at least one D-amino acid.

The variant protein may be expressed from an isolated DNA sequence encoding the variant protein. "Recombinant" is defined as a peptide or nucleic acid produced by the processes of genetic engineering. It should be noted that it is well-known in the art that, due to the redundancy in the genetic code, individual nucleotides can be readily exchanged in a codon and still result in an identical amino acid sequence. The terms "protein," "peptide" and "polypeptide" are used interchangeably herein.

The present disclosure provides methods of treating a disease in a mammal by administering an expression vector to a cell or patient. For the gene therapy methods, a person having ordinary skill in the art of molecular biology and gene therapy would be able to determine, without undue experimentation, the appropriate dosages and routes of administration of the expression vector used in the novel methods of the present disclosure.

According to one embodiment, the cells are transformed or otherwise genetically modified *in vivo.* The cells from the mammalian recipient are transformed *(i.e.,* transduced or transfected) *in vivo* with a vector containing exogenous genetic material for expressing a heterologous (e.g., recombinant) gene encoding a therapeutic agent and the therapeutic agent is delivered *in situ.*

As used herein, "exogenous genetic material" refers to a nucleic acid or an oligonucleotide, either natural or synthetic, that is not naturally found in the cells; or if it is naturally found in the cells, it is not transcribed or expressed at biologically significant levels by the cells. Thus, "exogenous genetic material" includes, for example, a non-naturally occurring nucleic acid that can be transcribed into anti-sense RNA, as well as a "heterologous gene" *(i.e.,* a gene encoding a protein which is not expressed or is expressed at biologically insignificant levels in a naturally-occurring cell of the same type).

In the certain embodiments, the mammalian recipient has a condition that is amenable to gene replacement therapy. As used herein, "gene replacement therapy" refers to administration to the recipient of exogenous genetic material encoding a therapeutic agent and subsequent expression of the administered genetic material *in situ.* Thus, the phrase "condition amenable to gene replacement therapy" embraces conditions such as genetic diseases (*i.e.,* a disease condition that is attributable to one or more gene defects), acquired pathologies (*i.e.,* a pathological condition which is not attributable to an inborn defect), cancers and prophylactic processes (*i.e.,* prevention of a disease or of an undesired medical condition). Accordingly, as used herein, the term "therapeutic agent" refers to any agent or material, which has a beneficial effect on the mammalian recipient. Thus, "therapeutic agent" embraces both therapeutic and prophylactic molecules having nucleic acid (*e.g*., antisense RNA) and/or protein components.

Alternatively, the condition amenable to gene replacement therapy is a prophylactic process, *i.e.,* a process for preventing disease or an undesired medical condition. Thus, the instant disclosure embraces a cell expression system for delivering a therapeutic agent that has a prophylactic function (*i.e.,* a prophylactic agent) to the mammalian recipient.

In summary, the term "therapeutic agent" includes, but is not limited to, agents associated with the conditions listed above, as well as their functional equivalents. As used herein, the term "functional equivalent" refers to a molecule (*e.g.,* a peptide or protein) that has the same or an improved beneficial effect on the mammalian recipient as the therapeutic agent of which is it deemed a functional equivalent.

The above-disclosed therapeutic agents and conditions amenable to gene replacement therapy are merely illustrative and are not intended to limit the scope of the instant disclosure. The selection of a suitable therapeutic agent for treating a known condition is deemed to be within the scope of one of ordinary skill of the art without undue experimentation.

### AAV Vectors

In one embodiment, a viral vector of the disclosure is an AAV vector. An "AAV" vector refers to an adeno-associated virus, and may be used to refer to the naturally occurring wild-type virus itself or derivatives thereof. The term covers all subtypes, serotypes and pseudotypes, and both naturally occurring and recombinant forms, except where required otherwise. As used herein, the term "serotype" refers to an AAV which is identified by and distinguished from other AAVs based on capsid protein reactivity with defined antisera, e.g., there are eight known serotypes of primate AAVs, AAV-1 to AAV-8. For example, serotype AAV2 is used to refer to an AAV which contains capsid proteins encoded from the cap gene of AAV2 and a genome containing 5' and 3' ITR sequences from the same AAV2 serotype. As used herein, for example, rAAV may be used to refer an AAV having both capsid proteins and 5'-3' ITRs from the same serotype or it may refer to an AAV having capsid proteins from one serotype and 5'-3' ITRs from a different AAV serotype, *e.g*., capsid from AAV serotype 2 and ITRs from AAV serotype 5. For each example illustrated herein the description of the vector design and production describes the serotype of the capsid and 5'-3' ITR sequences. The abbreviation "rAAV" refers to recombinant adeno-associated virus, also referred to as a recombinant AAV vector (or "rAAV vector").

An "AAV virus" or "AAV viral particle" refers to a viral particle composed of at least one AAV capsid protein (preferably by all of the capsid proteins of a wild-type AAV) and an encapsidated polynucleotide. If the particle comprises heterologous polynucleotide (*i.e.,* a polynucleotide other than a wild-type AAV genome such as a transgene to be delivered to a mammalian cell), it is typically referred to as "rAAV".

In one embodiment, the AAV expression vectors are constructed using known techniques to at least provide as operatively linked components in the direction of transcription, control elements including a transcriptional initiation region, the DNA of interest and a transcriptional termination region. The control elements are selected to be functional in a mammalian cell. The resulting construct which contains the operatively linked components is flanked (5' and 3') with functional AAV ITR sequences.

By "adeno-associated virus inverted terminal repeats" or "AAV ITRs" is meant the art-recognized regions found at each end of the AAV genome which function together in cis as origins of DNA replication and as packaging signals for the virus. AAV ITRs, together with the AAV rep coding region, provide for the efficient excision and rescue from, and integration of a nucleotide sequence interposed between two flanking ITRs into a mammalian cell genome.

The nucleotide sequences of AAV ITR regions are known. As used herein, an "AAV ITR" need not have the wild-type nucleotide sequence depicted, but may be altered, *e.g.,* by the insertion, deletion or substitution of nucleotides. Additionally, the AAV ITR may be derived from any of several AAV serotypes, including without limitation, AAV1, AAV2, AAV3, AAV4, AAV5, AAV7, etc. Furthermore, 5' and 3' ITRs which flank a selected nucleotide sequence in an AAV vector need not necessarily be identical or derived from the same AAV serotype or isolate, so long as they function as intended, *i.e.,* to allow for excision and rescue of the sequence of interest from a host cell genome or vector, and to allow integration of the heterologous sequence into the recipient cell genome when AAV Rep gene products are present in the cell.

In one embodiment, AAV ITRs can be derived from any of several AAV serotypes, including without limitation, AAV1, AAV2, AAV3, AAV4, AAV5, AAV7, etc. Furthermore, 5' and 3' ITRs which flank a selected nucleotide sequence in an AAV expression vector need not necessarily be identical or derived from the same AAV serotype or isolate, so long as they function as intended, *i.e.,* to allow for excision and rescue of the sequence of interest from a host cell genome or vector, and to allow integration of the DNA molecule into the recipient cell genome when AAV Rep gene products are present in the cell.

In one embodiment, AAV capsids can be derived from AAV2. Suitable DNA molecules for use in AAV vectors will be less than about 5 kilobases (kb), less than about 4.5 kb, less than about 4kb, less than about 3.5 kb, less than about 3 kb, less than about 2.5 kb in size and are known in the art.

In one embodiment, the selected nucleotide sequence is operably linked to control elements that direct the transcription or expression thereof in the subject *in vivo.* Such control elements can comprise control sequences normally associated with the selected gene. Alternatively, heterologous control sequences can be employed. Useful heterologous control sequences generally include those derived from sequences encoding mammalian or viral genes. Examples include, but are not limited to, the SV40 early promoter, mouse mammary tumor virus LTR promoter; adenovirus major late promoter (Ad MLP); a herpes simplex virus (HSV) promoter, a cytomegalovirus (CMV) promoter such as the CMV immediate early promoter region (CMVIE), a rous sarcoma virus (RSV) promoter, pol II promoters, pol III promoters, synthetic promoters, hybrid promoters, and the like. In addition, sequences derived from nonviral genes, such as the murine metallothionein gene, will also find use herein. Such promoter sequences are commercially available from, *e.g.,* Stratagene (San Diego, Calif.).

In one embodiment, both heterologous promoters and other control elements, such as CNS-specific and inducible promoters, enhancers and the like, will be of particular use. Examples of heterologous promoters include the CMV promoter. Examples of CNS-specific promoters include those isolated from the genes from myelin basic protein (MBP), glial fibrillary acid protein (GFAP), and neuron specific enolase (NSE). Examples of inducible promoters include DNA responsive elements for ecdysone, tetracycline, hypoxia and aufin.

In one embodiment, the AAV expression vector which harbors the DNA molecule of interest bounded by AAV ITRs, can be constructed by directly inserting the selected sequence(s) into an AAV genome which has had the major AAV open reading frames ("ORFs") excised therefrom. Other portions of the AAV genome can also be deleted, so long as a sufficient portion of the ITRs remain to allow for replication and packaging functions. Such constructs can be designed using techniques well known in the art.

Alternatively, AAV ITRs can be excised from the viral genome or from an AAV vector containing the same and fused 5' and 3' of a selected nucleic acid construct that is present in another vector using standard ligation techniques. For example, ligations can be accomplished in 20 mM Tris-Cl pH 7.5, 10 mM MgCl₂, 10 mM DTT, 33 µg/ml BSA, 10 mM-50 mM NaCl, and either 40 uM ATP, 0.01-0.02 (Weiss) units T4 DNA ligase at 0°C (for "sticky end" ligation) or 1 mM ATP, 0.3-0.6 (Weiss) units T4 DNA ligase at 14°C (for "blunt end" ligation). Intermolecular "sticky end" ligations are usually performed at 30-100 µg/ml total DNA concentrations (5-100 nM total end concentration). AAV vectors which contain ITRs.

Additionally, chimeric genes can be produced synthetically to include AAV ITR sequences arranged 5' and 3' of one or more selected nucleic acid sequences. Preferred codons for expression of the chimeric gene sequence in mammalian CNS cells can be used. The complete chimeric sequence is assembled from overlapping oligonucleotides prepared by standard methods.

In order to produce rAAV virions, an AAV expression vector is introduced into a suitable host cell using known techniques, such as by transfection. A number of transfection techniques are generally known in the art. See, *e.g.,* Sambrook et al. (1989) Molecular Cloning, a laboratory manual, Cold Spring Harbor Laboratories, New York. Particularly suitable transfection methods include calcium phosphate co-precipitation, direct microinjection into cultured cells, electroporation, liposome mediated gene transfer, lipid-mediated transduction, and nucleic acid delivery using high-velocity microprojectiles.

In one embodiment, suitable host cells for producing rAAV virions include microorganisms, yeast cells, insect cells, and mammalian cells, that can be, or have been, used as recipients of a heterologous DNA molecule. The term includes the progeny of the original cell which has been transfected. Thus, a "host cell" as used herein generally refers to a cell which has been transfected with an exogenous DNA sequence. Cells from the stable human cell line, 293 (readily available through, e.g., the American Type Culture Collection under Accession Number ATCC CRL1573) can be used in the practice of the present disclosure. Particularly, the human cell line 293 is a human embryonic kidney cell line that has been transformed with adenovirus type-5 DNA fragments, and expresses the adenoviral E1a and Elb genes. The 293 cell line is readily transfected, and provides a particularly convenient platform in which to produce rAAV virions.

By "AAV rep coding region" is meant the art-recognized region of the AAV genome which encodes the replication proteins Rep 78, Rep 68, Rep 52 and Rep 40. These Rep expression products have been shown to possess many functions, including recognition, binding and nicking of the AAV origin of DNA replication, DNA helicase activity and modulation of transcription from AAV (or other heterologous) promoters. The Rep expression products are collectively required for replicating the AAV genome. Suitable homologues of the AAV rep coding region include the human herpesvirus 6 (HHV-6) rep gene, which is also known to mediate AAV2 DNA replication.

By "AAV cap coding region" is meant the art-recognized region of the AAV genome which encodes the capsid proteins VP1, VP2, and VP3, or functional homologues thereof. These Cap expression products supply the packaging functions which are collectively required for packaging the viral genome.

In one embodiment, AAV helper functions are introduced into the host cell by transfecting the host cell with an AAV helper construct either prior to, or concurrently with, the transfection of the AAV expression vector. AAV helper constructs are thus used to provide at least transient expression of AAV rep and/or cap genes to complement missing AAV functions that are necessary for productive AAV infection. AAV helper constructs lack AAV ITRs and can neither replicate nor package themselves. These constructs can be in the form of a plasmid, phage, transposon, cosmid, virus, or virion. A number of AAV helper constructs have been described, such as the commonly used plasmids pAAV/Ad and pIM29+45 which encode both Rep and Cap expression products. A number of other vectors have been described which encode Rep and/or Cap expression products.

Methods of delivery of viral vectors include injecting the AAV into the CSF. Generally, rAAV virions may be introduced into cells of the CNS using either *in vivo* or *in vitro* transduction techniques. If transduced *in vitro,* the desired recipient cell will be removed from the subject, transduced with rAAV virions and reintroduced into the subject. Alternatively, syngeneic or xenogeneic cells can be used where those cells will not generate an inappropriate immune response in the subject.

Suitable methods for the delivery and introduction of transduced cells into a subject have been described. For example, cells can be transduced *in vitro* by combining recombinant AAV virions with CNS cells *e.g.,* in appropriate media, and screening for those cells harboring the DNA of interest can be screened using conventional techniques such as Southern blots and/or PCR, or by using selectable markers. Transduced cells can then be formulated into pharmaceutical compositions, described more fully below, and the composition introduced into the subject by various techniques, such as by grafting, intramuscular, intravenous, subcutaneous and intraperitoneal injection.

In one embodiment, pharmaceutical compositions will comprise sufficient genetic material to produce a therapeutically effective amount of the nucleic acid of interest, *i.e.,* an amount sufficient to reduce or ameliorate symptoms of the disease state in question or an amount sufficient to confer the desired benefit. The pharmaceutical compositions will also contain a pharmaceutically acceptable excipient. Such excipients include any pharmaceutical agent that does not itself induce the production of antibodies harmful to the individual receiving the composition, and which may be administered without undue toxicity. Pharmaceutically acceptable excipients include, but are not limited to, sorbitol, Tween80, and liquids such as water, saline, glycerol and ethanol. Pharmaceutically acceptable salts can be included therein, for example, mineral acid salts such as hydrochlorides, hydrobromides, phosphates, sulfates, and the like; and the salts of organic acids such as acetates, propionates, malonates, benzoates, and the like. Additionally, auxiliary substances, such as wetting or emulsifying agents, pH buffering substances, and the like, may be present in such vehicles. A thorough discussion of pharmaceutically acceptable excipients is available in Remington's Pharmaceutical Sciences (Mack Pub. Co., N.J. 1991).

As is apparent to those skilled in the art in view of the teachings of this specification, an effective amount of viral vector which must be added can be empirically determined. Administration can be effected in one dose, continuously or intermittently throughout the course of treatment. Methods of determining the most effective means and dosages of administration are well known to those of skill in the art and will vary with the viral vector, the composition of the therapy, the target cells, and the subject being treated. Single and multiple administrations can be carried out with the dose level and pattern being selected by the treating physician.

It should be understood that more than one transgene could be expressed by the delivered viral vector. Alternatively, separate vectors, each expressing one or more different transgenes, can also be delivered to the CNS as described herein. Furthermore, it is also intended that the viral vectors delivered by the methods of the present disclosure be combined with other suitable compositions and therapies.

### Methods for Introducing Genetic Material into Cells

The exogenous genetic material (*e.g*., a cDNA encoding one or more therapeutic proteins) is introduced into the cell ex vivo or *in vivo* by genetic transfer methods, such as transfection or transduction, to provide a genetically modified cell. Various expression vectors *(i.e.,* vehicles for facilitating delivery of exogenous genetic material into a target cell) are known to one of ordinary skill in the art.

As used herein, "transfection of cells" refers to the acquisition by a cell of new genetic material by incorporation of added DNA. Thus, transfection refers to the insertion of nucleic acid into a cell using physical or chemical methods. Several transfection techniques are known to those of ordinary skill in the art including: calcium phosphate DNA co-precipitation; DEAE-dextran; electroporation; cationic liposome-mediated transfection; and tungsten particle-faciliated microparticle bombardment. Strontium phosphate DNA co-precipitation is another possible transfection method.

In contrast, "transduction of cells" refers to the process of transferring nucleic acid into a cell using a DNA or RNA virus. A RNA virus (*i.e.,* a retrovirus) for transferring a nucleic acid into a cell is referred to herein as a transducing chimeric retrovirus. Exogenous genetic material contained within the retrovirus is incorporated into the genome of the transduced cell. A cell that has been transduced with a chimeric DNA virus (*e.g.*, an adenovirus carrying a cDNA encoding a therapeutic agent), will not have the exogenous genetic material incorporated into its genome but will be capable of expressing the exogenous genetic material that is retained extrachromosomally within the cell.

Typically, the exogenous genetic material includes the heterologous gene (usually in the form of a cDNA comprising the exons coding for the therapeutic protein) together with a promoter to control transcription of the new gene. The promoter characteristically has a specific nucleotide sequence necessary to initiate transcription. Optionally, the exogenous genetic material further includes additional sequences (*i.e.,* enhancers) required to obtain the desired gene transcription activity. For the purpose of this discussion an "enhancer" is simply any non-translated DNA sequence which works contiguous with the coding sequence (in cis) to change the basal transcription level dictated by the promoter. The exogenous genetic material may introduced into the cell genome immediately downstream from the promoter so that the promoter and coding sequence are operatively linked so as to permit transcription of the coding sequence. A retroviral expression vector may include an exogenous promoter element to control transcription of the inserted exogenous gene. Such exogenous promoters include both constitutive and inducible promoters.

Naturally-occurring constitutive promoters control the expression of essential cell functions. As a result, a gene under the control of a constitutive promoter is expressed under all conditions of cell growth. Exemplary constitutive promoters include the promoters for the following genes which encode certain constitutive or "housekeeping" functions: hypoxanthine phosphoribosyl transferase (HPRT), dihydrofolate reductase (DHFR), adenosine deaminase, phosphoglycerol kinase (PGK), pyruvate kinase, phosphoglycerol mutase, the actin promoter, and other constitutive promoters known to those of skill in the art. In addition, many viral promoters function constitutively in eucaryotic cells. These include: the early and late promoters of SV40; the long terminal repeats (LTRs) of Moloney Leukemia Virus and other retroviruses; and the thymidine kinase promoter of Herpes Simplex Virus, among many others. Accordingly, any of the above-referenced constitutive promoters can be used to control transcription of a heterologous gene insert.

Genes that are under the control of inducible promoters are expressed only or to a greater degree, in the presence of an inducing agent, (*e.g.,* transcription under control of the metallothionein promoter is greatly increased in presence of certain metal ions). Inducible promoters include responsive elements (REs) which stimulate transcription when their inducing factors are bound. For example, there are REs for serum factors, steroid hormones, retinoic acid and cyclic AMP. Promoters containing a particular RE can be chosen in order to obtain an inducible response and in some cases, the RE itself may be attached to a different promoter, thereby conferring inducibility to the recombinant gene. Thus, by selecting the appropriate promoter (constitutive versus inducible; strong versus weak), it is possible to control both the existence and level of expression of a therapeutic agent in the genetically modified cell. If the gene encoding the therapeutic agent is under the control of an inducible promoter, delivery of the therapeutic agent *in situ* is triggered by exposing the genetically modified cell *in situ* to conditions for permitting transcription of the therapeutic agent, *e.g.,* by intraperitoneal injection of specific inducers of the inducible promoters which control transcription of the agent. For example, *in situ* expression by genetically modified cells of a therapeutic agent encoded by a gene under the control of the metallothionein promoter, is enhanced by contacting the genetically modified cells with a solution containing the appropriate (*i.e.,* inducing) metal ions *in situ.*

Accordingly, the amount of therapeutic agent that is delivered *in situ* is regulated by controlling such factors as: (1) the nature of the promoter used to direct transcription of the inserted gene, *(i.e.,* whether the promoter is constitutive or inducible, strong or weak); (2) the number of copies of the exogenous gene that are inserted into the cell; (3) the number of transduced/transfected cells that are administered (*e.g.,* implanted) to the patient; (4) the size of the implant (*e.g.,* graft or encapsulated expression system); (5) the number of implants; (6) the length of time the transduced/transfected cells or implants are left in place; and (7) the production rate of the therapeutic agent by the genetically modified cell. Selection and optimization of these factors for delivery of a therapeutically effective dose of a particular therapeutic agent is deemed to be within the scope of one of ordinary skill in the art without undue experimentation, taking into account the above-disclosed factors and the clinical profile of the patient.

In addition to at least one promoter and at least one heterologous nucleic acid encoding the therapeutic agent, the expression vector may include a selection gene, for example, a neomycin resistance gene, for facilitating selection of cells that have been transfected or transduced with the expression vector. Alternatively, the cells are transfected with two or more expression vectors, at least one vector containing the gene(s) encoding the therapeutic agent(s), the other vector containing a selection gene. The selection of a suitable promoter, enhancer, selection gene and/or signal sequence (described below) is deemed to be within the scope of one of ordinary skill in the art without undue experimentation.

The therapeutic agent can be targeted for delivery to an extracellular, intracellular or membrane location. If it is desirable for the gene product to be secreted from the cells, the expression vector is designed to include an appropriate secretion "signal" sequence for secreting the therapeutic gene product from the cell to the extracellular milieu. If it is desirable for the gene product to be retained within the cell, this secretion signal sequence is omitted. In a similar manner, the expression vector can be constructed to include "retention" signal sequences for anchoring the therapeutic agent within the cell plasma membrane. For example, all membrane proteins have hydrophobic transmembrane regions, which stop translocation of the protein in the membrane and do not allow the protein to be secreted. The construction of an expression vector including signal sequences for targeting a gene product to a particular location is deemed to be within the scope of one of ordinary skill in the art without the need for undue experimentation.

### Example 1

### Changes in the Progression of Amyloid Deposition

This Example studied changes in the progression of amyloid deposition in app/ps mice after overexpression of different ApoE isoforms through intraventricular injection of an adeno-associated virus serotype 4 (AAV4).

The epsilon 4 allele of ApoE (ApoE ε4) is the first genetic risk factor for Alzheimer disease (AD), whereas inheritance of the rare epsilon 2 allele of ApoE (ApoE ε2) reduces this risk by about half. However, despite the discovery of these strong genetic clues almost 17 years ago, the mechanisms whereby ApoE confer risk remains uncertain.

In order to decipher how the different ApoE isoforms (ApoE ε2, ε3 and ε4) impact the formation and stability of fibrillar amyloid plaques, AAV4 vectors coding for each ApoE isoform were injected into the ventricle of 7 month-old APP/PS mice. Using in vivo multiphoton imaging, populations of amyloid deposits were tracked at baseline and after exposure to ApoE over a two-month interval, thus allowing a dynamic view of amyloidosis progression in a living animal.

The kinetic of amyloid plaque deposition was observed to be variable according to each isoform, so that ApoE ε4 injected mice had a 38% increase of senile plaques whereas mice treated with ApoE ε2 presented a 15% decrease of the number of amyloid deposits compared to ApoE ε3 after 2 months. The post-mortem analysis confirmed these results and revealed the presence of human ApoE proteins decorating plaques in the cortex, reflecting a large diffusion of the protein throughout the parenchyma and its focal accumulation where Aβ peptides are deposited. It is important to note that this increased content of ApoE ε4 protein was also associated with a more severe synapse loss around the amyloid deposits.

Overall, the present data demonstrated that over-production of different ApoE isoforms was able to influence the progression of the disease and could modulate the extent of synapse loss, one of the parameters that correlates best with cognitive impairment in AD patients

### 1. Intraventricular injection of AA V4-ApoE led to a stable expression of huApoE and a sustained detection of recombinant human ApoE (huApoE) protein in the brain.

Briefly, GFP and huApoE were immunodetected in APP/PS mice injected with AAV4 vectors. GFP signal could be observed into the entire ventricle area (upper panel) and in the cells lining the ventricle, as well as human APOE.

In order to evaluate the present approach, AAV4-Venus (control), -ApoE2, -ApoE3 and -ApoE4 were injected in the ventricle of wild-type mice. Two months after injection, human ApoE proteins could be detected in the cortical parenchyma around amyloid deposits (note the 3H1 antibody; only nonspecific background was observed in AAV4-GFP injected mice). Thus, a significant level of human ApoE was detected by ELISA in the brain, and immunohistological stainings for Venus and ApoE confirmed the expression of the different transgenes by the cells lining the ventricle.

qRT-PCR experiments were performed in order to evaluate the mRNA levels of the transgene. A standard curve lowed us to determine the concentrations of huApoE mRNA according to the level of endogenous GAPDH. Samples from mice that were exposed for 2 or 5 months were included. An ELISA assay designed to specifically detect human APOE was performed on brain homogenates (**Fig. 1A**). Low levels of recombinant proteins could be detected in AAV4-APOE injected mice compared with AAV4-GFP treated animals, as quantified by ELISA specific for human APOE (**Fig. 1B**) and confirmed by Western blot.

### 2. Overexpression of each isoform of APOE differentially affects the progression of the amyloidosis.

*In vivo* two-photon imaging was used to follow amyloid deposition over time in a living animal. Briefly, APP/PS mice (7 month old) were stereotactically injected with AAV4 vectors coding for ApoE2, ApoE3, ApoE4 and Venus. After 1 week, a cranial window was implanted and amyloid deposits were imaged over time after craniotomy. After 2 months, the animals were sacrificed and post-mortem analyses were performed.

2-photon images of APP/PS mice injected with AAV4-ApoE2, AAV4-ApoE3 or AAV4-ApoE4 were prepared. Amyloid plaques could be detected after intraperitoneal injection of Methoxy-XO4 (5mg/kg) and Texas Red dextran (70,000 Da molecular weight; 12.5 mg/ml in sterile PBS) was injected into a lateral tail vein to provide a fluorescent angiogram. Images were taken one week (=T0), one month and two months after injection. The same fields were captured over time to follow the progression of the lesions. Few new amyloid deposits appeared whereas few of them could not be detected anymore over a two-month period of time.

A complete analysis of in vivo images shows that the number of amyloid deposits increases significantly more rapidly in AAV4-ApoE4 injected APP/PS mice compared with both AAV4-ApoE3 and AAV4-Venus treated animals. By contrast, a small but significant decrease density of plaques is measured when AAV4-ApoE2 was used (**Fig. 2**). A trend toward bigger plaques is observed in APP/PS mice injected with AAV4-ApoE4 (p<0.06), but overall the size of the plaques remains constant. The summarized data of *in vivo* imaging shows that overexpression of each APOE isoform differentially affect the progression of amyloid deposition in vivo. Injection of AAV4-ApoE2 leads to a slight decrease of amyloid density over times, whereas injection of AAV4-ApoE4 aggravates the amyloidosis.

### 3. The size of amyloid plaques vary according to each ApoE isoform.

*In vivo* two-photon imaging allowed the following of the changes of the size of each amyloid deposit over a period of 2 months. The size of plaques may remain stable , increase or decrease over time. The distributions of the size ratios between T1/T0 and T2/T1 show that there is a shift towards bigger amyloid plaques in mice injected with an AAV4-ApoE4 compared with the other groups (**Fig. 3**).

### 4. Post-mortem evaluation of amyloid load confirms the effects of ApoE2 and ApoE4 on amyloid deposition.

Two months after AAV4 injection, the post-mortem stereological evaluation revealed that AAV4-ApoE4 injected animals have a higher density of amyloid plaques in the cortex, whereas no difference could be detected between the other groups (**Fig. 4A**). This increased number of amyloid deposits is observed when plaques were labeled with ThioS or Bam10. However, no change in the ratio between Bam10 and ThioS was detected. Five months after injection, the effects of each ApoE isoform are more pronounced compared with two months (**Fig. 4B**). A significant increased density of deposits was observed when mice were injected with AAV4-ApoE4 whereas an inverse effect was detected with ApoE2. Again, no change in the ratio between Bam10 and ThioS awes detected.

### 5. Each ApoE is form differentially affects synaptic density around amyloid deposits.

Array tomography was used to precisely determine the density of pre- and post-synaptic elements around amyloid deposits. This new imaging method offers capabilities for high-resolution imaging of tissue molecular architecture. Array tomography is based on ultrathin sectioning of the specimen (70nm), immunostaining and 3D reconstruction. Representative images of array tomography samples stained for amyloid plaque and the post-synaptic maker PSD95. Array tomography images show that a decreased number of the post-synaptic marker PSD95 is observed around the amyloid deposits, but this effect is abolished far from plaques. The quantification of pre- (synapsin-1) and post-synaptic markers in the vicinity or far from plaques was determined in each group of mice injected with an AAV4 (**Fig. 5A-D**). The extensive quantification of pre- and post-synaptic elements confirmed that a decreased density of synapsin 1 and PSD95 was associated with amyloid plaques, this effect being dramatically amplified when ApoE4 was overexpressed in the brain of APP/PS1 mice (**Fig. 5C, Fig. 5D**). Overexpression of ApoE4 is associated with an increased spine loss compared with the other groups in the vicinity of amyloid deposits. On the contrary, the density of synapsin puncta is higher in ApoE2 treated animals around plaques.

### Conclusion

Intraventricular injections of AAV virus serotype 4 led to a sustained and chronic over-production of soluble recombinant proteins throughout the cerebral parenchyma. Overexpression of ApoE2, ApoE3 and ApoE4 differentially affected the course of the pathology in APP/PS mice, so that the progression of amyloid load was significantly increased when ApoE4 is injected compared with ApoE3. Conversely, ApoE2 was associated with protective effects and few amyloid deposits are not detectable anymore two months after injection. The post-mortem immunohistological analysis confirmed the adverse effect of ApoE4. Sustained over-production of ApoE4 exacerbated the synapse loss observed around amyloid deposits compared with ApoE3, whereas ApoE2 had a mild effect. The present study demonstrated a direct connection between ApoE isoforms, amyloidosis progression and synapse loss *in vivo.*

### Example 2

### Treating Central Nervous System Disorders via Cerebral Spinal Fluid (CSF) in Large Mammals

In order to achieve gene therapy for brain disorders, such as Alzheimer's disease, it needed to be determined whether long-term, steady-state levels of therapeutic enzymes could be achieved in a mammal. It was discovered that ependymal cells (cells that lie the ventricles in the brain) can be transduced and secrete a targeted enzyme into the cerebral spinal fluid (CSF). It was determined that adeno-associated virus (AAV4) can transduce the ependyma in a mouse model with high efficiency. (Davidson et al, PNAS, 28:3428-3432, 2000.) In mice there was a normalization of stored substrate levels in disease brain after AAV4 treatment.

It was investigated whether global delivery of a vector could be effectively performed in order to achieve steady-state levels of enzyme in the CSF. First, a vector needed to be found that could transduce ependymal cells (cells that line the ventricles) in the brain of larger mammals. Studies were performed in a dog model of LINCL and a non-human primate model of LINCL. The LINCL dogs are normal at birth, but develop neurological signs around 7 months, testable cognitive deficits at ∼ 5-6 months, seizures at 10-11 months, and progressive visual loss.

An adeno-associated virus (AAV) was selected as the vector because of its small size (20 nm), most of its genetic material can be removed ("gutted") so that no viral genes are present, and so that it is replication incompetent. It was previously tested whether adeno-associated virus type 4 (AAV4) vectors could mediate global functional and pathological improvements in a murine model of mucopolysaccharidosis type VII (MPS VII) caused by beta-glucuronidase deficiency (Liu et al., J. Neuroscience, 25(41):9321-9327, 2005). Recombinant AAV4 vectors encoding beta-glucuronidase were injected unilaterally into the lateral ventricle of MPS VII mice with established disease. Transduced ependyma expressed high levels of recombinant enzyme, with secreted enzyme penetrating cerebral and cerebellar structures, as well as the brainstem. Immunohistochemical studies revealed close association of recombinant enzyme and brain microvasculature, indicating that beta-glucuronidase reached brain parenchyma via the perivascular spaces lining blood vessels. Aversive associative learning was tested by context fear conditioning. Compared with age-matched heterozygous controls, affected mice showed impaired conditioned fear response and context discrimination. This behavioral deficit was reversed 6 weeks after gene transfer in AAV4 beta-glucuronidase-treated MPS VII mice. The data show that ependymal cells can serve as a source of enzyme secretion into the surrounding brain parenchyma and CSF.

Surprisingly, however, when these studies were extended to large mammals (i.e., dogs and non-human primates), the AAV4 vectors were not effective in targeting the ependyma in these animals. Instead, an AAV2 vector needed to be used. Briefly, rAAV2 was generated encoding TPP1 (AAV2-CLN2), and injected intraventricularly to transduce ependyma (Liu et al., J. Neuroscience, 25(41):9321-9327, 2005). TPP1 is the enzyme deficient in LINCL. The data indicated that ependymal transduction in NHP brain resulted in a significant increase of enzyme in CSF. The results indicated elevated levels of TPP1 activity in various brain regions, where the vertical axis show % control of activity (**Figure 7**).

In the first dog that was treated, the delivery of vector was suboptimal, but still exhibited CLN2 activity in the brain. Subsequent dogs underwent ICV delivery with stereotaxy. It was found that the cognitive abilities of the treated dogs were significantly improved over a non-treated dog, as measured by T-maze performance (**Figure 8**). Further, the effects of ICV delivery of AAV2-CLN2 in the dog model of LINCL were very pronounced. In the untreated (-/-) animal, large ventricles are present, whereas the brains of the untreated control and the treated animals did not exhibit ventricles. Following delivery of AAV.TPP1 to ventricles of LINCL dogs, detectable enzyme activity was noted in various brain regions, including the cerebellum and upper spinal cord. In two living additional affected dogs, brain atrophy was significantly attenuated, longevity was increased and cognitive function was improved. Finally, in NHP, we show that this method can achieve TPP1 activity levels 2-5 fold above wildtype.

Several AAV vectors were generated and tested to determine the optimal combination of ITR and capsid. Five different combinations were produced, once it was determined that the AAV2 ITR was most effective: AAV2/1 (i.e., AAV2 ITR and AAV1 capsid), AAV2/2, AAV2/4, AAV2/5, and AAV2/8. It was discovered that AAV2/2 worked much better in the large mammals (dogs and NHP), followed by AAV2/8, AAV2/5, AAV2/1 and AAV2/4. This was quite surprising because the order of effectiveness of the viral vectors is the opposite of what was observed in mice.

Thus, the present work has shown that ventricular lining cells can be a source of recombinant enzyme in CSF for distribution throughout the brain, and that AAV2/2 is an effective vehicle for administering therapeutic agents, such as the gene encoding CLN2 (TPP1) in dogs and nonhuman primates.

### Example 3

### Human APOE Isoforms Delivered Via Gene Transfer Differentially Modulate Alzheimer's Disease By Affecting Amyloid Deposition, Clearance, And Neurotoxicity

Alzheimer's disease (AD) is the most frequent age-related neurodegenerative disorder and has become a major public health concern. Among the susceptibility genes associated with the late onset sporadic form of AD, the apolipoprotein E ε4 (*APOE* - gene; ApoE - protein) allele is by far the most significant genetic risk factor. The presence of one *APOE ε4* copy substantially increases the risk to develop the disease by a factor of 3 compared with the most common *APOE ε3* allele, whereas two copies lead to a 12-fold increase. Intriguingly, *APOE* ε2 has an opposite impact and is a protective factor, so that inheritance of this specific allele decreases the age-adjusted risk of AD by about a half compared to *APOE3*/*3.* The average age of onset of dementia also corresponds to these risk profiles, with *APOE4*/*4* carriers having an onset in their mid-60's and *APOE2*/*3* carriers in their early 90's, a shift of almost 3 decades, whereas *APOE3*/*3* individuals have an age of onset in between - in the mid 1970's.

The mechanism whereby ApoE impacts AD is controversial. The accumulation of Aβ containing senile plaques in the hippocampus and cortex of patients is believed to play a central role in AD, because all the known genes responsible for the rare autosomal dominant forms of the disease participate in the production of Aβ peptides. Interestingly, *APOE* genotype was shown to strongly affect the extent of amyloid deposition in patients with AD as well as the amount of neurotoxic soluble oligomeric Aβ detected in autopsy samples. ApoE isoforms have been suggested to differentially influence cerebrovascular integrity and affect the efflux of Aβ peptides through the blood brain barrier, thus modulating the buildup of amyloid aggregates around blood vessels (cerebral amyloid angiopathy or CAA). In addition, ApoE has also been implicated directly in neurodegeneration and in neuronal plasticity. The effects of ApoE2 have been relatively understudied in these contexts.

Genetically engineered animals expressing human *APOE2, -E3* and *-E4* have a similar rank order of amyloid burden as humans, consistent with the hypothesis that different ApoE isoforms impact plaque initiation and/or growth. However, further studies are needed to dissect mechanisms of ApoE mediated effects on existing amyloid deposits and on extant neurodegeneration. To overcome this gap in knowledge, we used a gene transfer approach in which adeno-associated virus vectors expressing the various *APOE* alleles (or GFP control) are injected into the lateral ventricle to primarily transduce the ependyma, which then act as a biological factory to deliver ApoE within the cerebrospinal and interstitial fluids. We then used intravital multiphoton microscopy to track the effects of various ApoE isoforms on plaque formation, growth, and in the case of ApoE2, dissolution, as well as *in vivo* microdialysis approaches to monitor ApoE and Aβ biochemical variables in the ISF, and array tomography to evaluate changes in Aβ-associated neurotoxicity. We found that ApoE isoforms impact the levels of soluble oligomeric Aβ in the ISF, the pace of Aβ fibrillization and deposition, the stability of amyloid deposits once formed, their clearance, and the extent of peri-plaque neurotoxic effects. Indeed, AD mice treated with ApoE4 show an enhanced amount of soluble Aβ, a higher density of fibrillar plaques, an exacerbation of synaptic element loss and an increased number of neuritic dystrophies around each deposit, whereas a relative protective effect was observed with ApoE2. These data support the hypothesis that *APOE* alleles mediate their effect on AD primarily through Aβ, and highlight ApoE as a therapeutic target.

### RESULTS

### Intraventricular injection of AAV4-APOE leads to stable APOE expression and to sustained production of human ApoE in the brain

Apolipoprotein E is a naturally secreted protein, produced mainly by astrocytes and microglial cells and can diffuse throughout the cerebral parenchyma. We took advantage of this property by injecting an AAV serotype 4 coding for *GFP* (control) or each *APOE* allele into the lateral cerebral ventricles of 7 month-old APP/PS1 mice. Considering the large cerebral areas affected by the characteristic lesions of AD, this strategy offered a great advantage compared with multiple intraparenchymal injections.

Two months after injection, transduced cells were detected in the choroid plexus and ependyma lining the ventricle, thus confirming the functionality of the AAV4 vectors. Using antibodies specific for each species, both human and murine ApoE proteins were also detected by ELISA (**Fig. 9A, 9B** **and** **15A**) and Western Blot. We observed that the concentration of human apolipoprotein E reached 20 µg/mg of total protein on average (**Fig. 9A**), representing about 10% of the endogenous murine apoE (**Fig. 9B**). The presence of this modest additional amount of human ApoE did not detectably alter the levels of endogenous murine apoE protein (**Fig. 15A**). A small but statistically significant decrease was observed between 2 and 5 months after the AAV4 injection (**Fig. 15B**). Nonetheless, the levels of human protein remained detectable compared with the control group, suggesting that AAV4-mediated transduction provided a platform for sustained production of the secreted recombinant protein throughout the parenchyma. Indeed, human ApoE proteins could be detected around amyloid deposits of APP/PS 1 mice throughout the cortical mantle, where endogenous murine apoE protein is known to accumulate.

Next, we assessed the presence of human ApoE in the interstitial fluid (ISF), an extracellular compartment that also contains highly biologically active Aβ soluble species. Because of the relatively small amount of ApoE detected in the entire brain lysate, we injected several *apoE* KO mice with each AAV4-APOE vector, and tracked the presence of the human protein using highly sensitive but non-species specific antibodies. Using a microdialysis technique, we confirmed the presence of ApoE in the ISF of *apoE* KO injected animals.

Overall, these data confirm that a single intracerebroventricular injection of AAV4 was sufficient to lead to sustained production of a protein of interest throughout the entire brain parenchyma and within the ISF, and that the ependyma/choroid plexus can be used as a "biological pump" to deliver potentially therapeutic proteins to the brain.

### Infusion of the ApoE isoforms differentially affect amyloid peptides and plaque deposition

APP/PS 1 mice were transduced with vectors expressing GFP or the various ApoE isoforms for 5 months before euthanasia. An analysis of the amyloid plaque load revealed that, after 5 months, a significant increase in the density of amyloid deposits was observed in the cortex of animals injected with the AAV4-APOE4 compared with those expressing APOE2. Plaque density in AAV4-GFP and AAV4-APOE3 treated mice were not different from one another at an intermediate level (**Fig. 16A**).

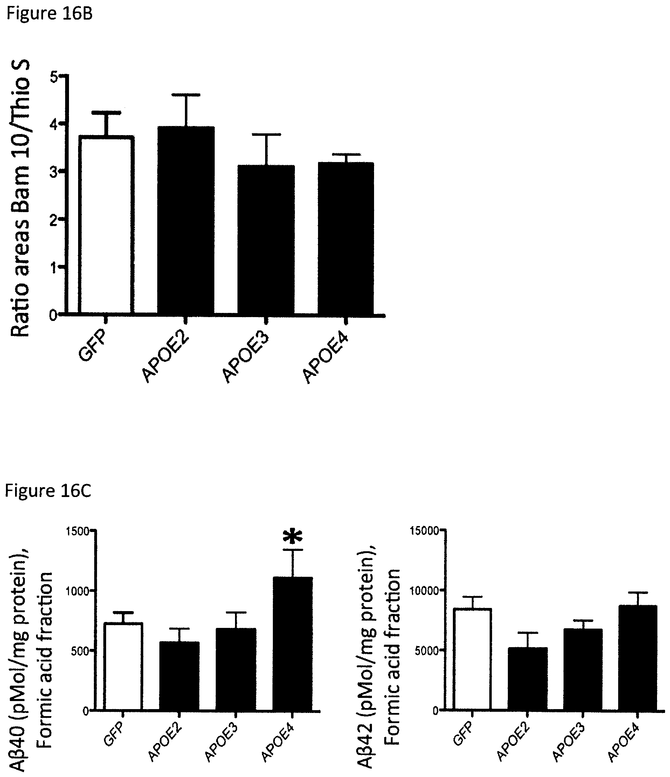

The concentrations of Aβ₄₀ and Aβ₄₂ peptides measured from the formic acid extracts mimicked the changes observed in the amyloid plaques content, so that an increased concentration of amyloid peptides was found in mice expressing the *APOE4* allele (**Fig. 16B**), and an opposite effect was detected with *APOE2* after 5 months. The content of Aβ₄₀ and Aβ₄₂ peptides in the TBS-soluble fraction was similarly affected by the injection of each AAV-APOE (**Fig. 16C**). In addition, the ratio between aggregated and soluble Aβ peptides remained unchanged by ApoE exposure, thus suggesting that overexpression of each distinct human ApoE isoform concomitantly modulates both the fibrillar and soluble amyloid species.

Overexpression of each ApoE isoform for only 2 months leads to smaller effects than observed in the 5 month study. Nevertheless, a significant increase in amyloid plaque density within the cortical area of AAV4-APOE4 injected mice was observed compared with the other experimental groups (**Fig. 16A**). This was paralleled by the amount of Aβ contained in the formic acid fraction (**Fig. 16C**), demonstrating a predominant effect of this specific variant. TBS-soluble Aβ_{40/42} species only showed a tendency to be lower or higher when AAV4-APOE2 or AAV4-APOE4, respectively, was expressed for 2 months (data not shown).

To determine if the presence of human ApoE isoforms might reflect an early change in the degree of fibrillization of Aβ, we also measured the ratio between robust immunostaining for Aβ using Bam10 (that labels all amyloid deposits) and Thio-S (that only stains the dense core) 2 months after injection. No change was detected among the 3 isoforms, suggesting that there was no differential effect on the distribution of the dense and diffuse amyloid deposits populations across the experimental groups in this time frame (**Fig. 16B**). These data indicate that a longer exposure to ApoE variants has stronger effects on amyloid deposition than shorter exposure.

It has been suggested that ApoE plays a role in Aβ transport across the blood-brain barrier. To test if exposure to the ApoE isoforms might modulate the efflux of Aβ peptides through the blood brain barrier, the concentration of Aβ₄₀ was measured in the plasma of each injected animal. We observed that the plasma content of human Aβ in both intracerebroventricularly injected AAV4-APOE3 and AAV4-APOE4 mice was lower compared with AAV4-APOE2 and AAV4-GFP (**Fig. 10D**). This suggests that both E3 and E4 variants help retain Aβ in the central nervous system compartment, consistent with the relative increased concentrations of Aβ in cerebral parenchyma observed and with previous data suggesting enhanced half-life of Aβ due to ApoE.

*APOE4* carriers are more susceptible to neurovascular dysfunction, and blood brain barrier breakdown was recently shown to be favored in APOE4 transgenic mice even in the absence of amyloid deposition. In order to assess if an intraventricular injection of an AAV4-APOE in APP/PS might compromise the integrity of the BBB, post-mortem staining with Prussian blue was performed. Despite the presence of few hemosiderin positive focal areas sparsely spread across the brain in all groups, no obvious differences were observed between any of the experimental groups of animals.

### Expression of ApoE isoforms modulates the kinetics of the progression of amyloidosis

ApoE4 was associated with an increased density of amyloid deposits, whereas the opposite effect was observed with ApoE2 after 5 months. This could reflect changes in the rates of amyloid β deposition, clearance, or both. To assess how the ApoE variants affect the dynamic progression of amyloidosis, we used *in vivo* two-photon imaging and followed the kinetics of amyloid plaque formation and clearance. Mice received an intraventricular injection with an AAV4 vectors at 7 months of age and a cranial window was implanted one week after injection in order to perform the first imaging session (T0). After 1 (T1) and 2 month(s) (T2), amyloid deposits were imaged in the same fields of view. Mice were euthanized for post-mortem analysis after the second imaging session.

The vast majority of amyloid deposits remained stable, although occasional new plaques could be detected in the small viewing volume over the two-month time period. Moreover, on rare occasion, a Methoxy-positive plaque that was imaged at the beginning of the experiment could not be detected after one or two month(s), suggesting that some plaques could be cleared. Over time, we observed an overall increase in the volumetric density of amyloid deposits, with the density at T2 on average 23% greater than of T1. The rate of amyloid progression was faster in ApoE4 treated APP/PS 1 mice, whereas ApoE2 exposed animals had a significantly reduced amyloid deposit density relative to GFP (0.66), ApoE3 (0.67) and ApoE4 (0.74) after 2 months (**Figs. 11A,11B**). Importantly, the ApoE2 changes reflect a decrease from baseline, showing directly, and for the first time, non-immune mediated active clearance of plaques. In contrast to data obtained from APOE transgenic animals, these results demonstrate that induction of a modest increase of the amount of ApoE can affect the ongoing amyloidogenic process even after amyloid deposition has already started.

We next assessed single amyloid plaque growth by measuring the ratio of the cross-sectional area of individual deposits between T1/T0 and T2/T1. Differences were detected among groups at T1 (ratio T1/T0), but not at T2 (ratio T2/T1, **Fig. 12**), suggesting that the presence of human ApoE variants mainly affects the plaque growth during the first month after exposure, but this parameter does not differ afterwards. In particular, the size of amyloid deposits grew significantly more in ApoE4 treated mice compared with both ApoE2 and ApoE3, suggesting that not only the number of plaques as well as their size was exacerbated by this allele. ApoE4 therefore affects both the seeding of Aβ peptides as well as the size of pre-existing plaques.

### Synaptic density around amyloid deposits is worsened by ApoE3 and ApoE4 isoforms compared to ApoE2

Synapse loss is a parameter that correlates best with cognitive impairment. We recently showed that the presence of ApoE4 is associated with higher levels of synaptic oligomeric Aβ in the brains of human AD patients and leads to significantly decreased synapse density around amyloid plaques compared to ApoE3 (R. M. Koffie et al., Apolipoprotein E4 effects in Alzheimer's disease are mediated by synaptotoxic oligomeric amyloid-beta. Brain 135, 2155 (Jul, 2012); T. Hashimoto et al., Apolipoprotein E, Especially Apolipoprotein E4, Increases the Oligomerization of Amyloid beta Peptide. J Neurosci 32, 15181 (Oct 24, 2012)). In addition, recent *in vitro* evidence demonstrated that ApoE4 failed to protect against Aβ induced synapse loss (M. Buttini et al., Modulation of Alzheimer-like synaptic and cholinergic deficits in transgenic mice by human apolipoprotein E depends on isoform, aging, and overexpression of amyloid beta peptides but not on plaque formation. J Neurosci 22, 10539 (Dec 15, 2002); A. Sen, D. L. Alkon, T. J. Nelson, Apolipoprotein E3 (ApoE3) but not ApoE4 protects against synaptic loss through increased expression of protein kinase C epsilon. J Biol Chem 287, 15947 (May 4, 2012)). We therefore hypothesized that a continuous and diffuse distribution of each ApoE isoform may not only differentially affect the kinetics of Aβ deposition and clearance in the brain of APP/PS mice, but also the integrity of synapses surrounding amyloid deposits.

The densities of pre- and post-synaptic elements (respectively synapsin-1 and PSD95) were determined using array tomography, a high-resolution technique based on immunofluorescence staining of ultrathin tissue sections (K. D. Micheva, S. J. Smith, Array tomography: a new tool for imaging the molecular architecture and ultrastructure of neural circuits. Neuron 55, 25 (Jul 5, 2007); R. M. Koffie et al., Oligomeric amyloid beta associates with postsynaptic densities and correlates with excitatory synapse loss near senile plaques. Proc Natl Acad Sci U S A 106, 4012 (Mar 10, 2009)). As amyloid oligomeric species were shown to be highly concentrated in the close vicinity of amyloid deposits, synapsin-1 and PSD95 puncta were quantified either far (> 50µm) or close (< 50µm) from plaques using previously established protocols (R. M. Koffie et al., Oligomeric amyloid beta associates with postsynaptic densities and correlates with excitatory synapse loss near senile plaques. Proc Natl Acad Sci U S A 106, 4012 (Mar 10, 2009)). We observed that the loss of pre-synaptic elements near plaques was exacerbated when either *APOE3* or *APOE4* was expressed, which was not the case after injection of AAV4-APOE2 or AAV4-GFP (**Fig. 13A**). By contrast, the density of post-synaptic puncta remained unchanged between GFP, ApoE2 and ApoE3 injected mice, whereas ApoE4 treated animals showed a significant loss of PSD95 around amyloid deposits, thus reinforcing the deleterious effect of ApoE4 on the neurotoxic effects of Aβ (**Fig. 13C**). When the density of synaptic elements was evaluated in areas located far from amyloid deposits (> 50µm), no difference could be detected between the groups, suggesting that there is no effect of the human ApoE variants *per se* on synaptic density, but an important effect of ApoE isoforms on Aβ induced neurotoxicity. The relative synaptic loss observed with ApoE3 and ApoE4 is therefore directly related to the presence of Aβ peptides surrounding each plaque (at a distance <50 µm from its edge).

As an additional neuropathological parameter, we also evaluated the number of neuritic dystrophies associated with amyloid deposits in AAV4 injected APP/PS 1 mice. In addition to a decreased spine density around them, senile plaques also cause a more general alteration of the neuropil with an increase of neurite curvature and the appearance of swollen dystrophies. These pathological changes are likely attributable to soluble oligomeric Aβ species that are enriched in a region within 50µm of the plaque surface. We observed that overexpression of ApoE4 exacerbates the formation of SMI312-positive neuritic dystrophies associated with amyloid deposits compared with GFP, ApoE2 and ApoE3 (**Fig. 13C**). This result confirms the observation that the ApoE4 isoform has the strongest effect and not only modulates plaque formation but also affects amyloid associated neurotoxicity.

### Human ApoE proteins modify the amount oligomeric Aβ species contained in the interstitial fluid in another mouse model of AD

We next addressed the question of whether the presence of different ApoE isoforms within the ISF may alter the amount of soluble amyloid species in that same extracellular compartment. We chose to inject another model of AD, the Tg2576 mice, in order to validate our previous findings in a different transgenic mouse line. Tg2576 mice overexpress the mutated form of APP containing the Swedish mutation and present a much milder phenotype than APP/PS1 mice at a given age. We injected cohorts of 16 to 18 month old animals, so that amyloid deposits were already present at the time of AAV4-APOE transduction. Three months after gene transfer, a microdialysis probe was inserted into the hippocampus and samples were collected to characterize early changes associated with each APOE variant within the ISF.

We observed that the concentration of Aβ oligomeric species measured using the specific 82E1/82E1 ELISA assay was significantly higher (by 42±7%) after injection of the AAV4-APOE4 compared with AAV4-APOE2 (**Fig. 14**), suggesting that the presence of ApoE may modulate the nature of amyloid aggregates in this extracellular compartment. Moreover, when the total Aβ₄₀ and Aβ₄₂ were assessed in the ISF, the same trends were observed but did not reach significance (**Fig. 17A**), suggesting that the presence of different ApoE isoforms in the ISF influences the aggregation state of amyloid peptides somewhat more than the total amount.

As expected, post-mortem biochemical analyses of brains from Tg2576 mice exposed to the various ApoE isoforms showed that the concentration of Aβ₄₂ in the formic acid fraction was significantly increased in ApoE4 treated animals (**Fig. 17B**), confirming in a second transgenic model our observations in APP/PS 1 mice.

Taken together, these biochemical measures suggest that ApoE expression in Tg2576 mice induce similar changes in amyloid biology as observed in APP/PS 1 mice. Importantly, an early change is observed in the content of oAβ within the ISF, where these neurotoxic species can directly interact with the synaptic terminals.

### DISCUSSION ---

The striking connection between inheritance of *APOE4* alleles increasing risk, and *APOE2* alleles having a dramatic opposite effect for the development of AD has led to multiple suggestions as to how this risk is mediated. ApoE has been implicated as an Aβ binding protein involved in Aβ clearance. However, studies in *apoE* knockout mice surprisingly reported that Aβ deposits were substantially lower in the absence of apoE. Replacement with human *APOE2, APOE3,* or *APOE4* led to increasing amyloid deposits in the same order as in AD patients, which was postulated to occur via an effect on plaque initiation or fibril formation. Alternative hypotheses focus on differential effects on neuritic outgrowth, or even propose that the effect of APOE genotype on Alzheimer disease phenotype is a consequence of another gene in genetic disequilibrium with *APOE* on chromosome 19.

Our data, derived from study of 2 different mouse models using an approach previously tested in the setting of lysosomal storage disease and Huntington disease, directly addressed these issues by using a combination of *in vivo* multiphoton imaging, standard quantitative immunohistopathology, array tomography studies of synaptic structure, and novel high molecular weight microdialysis approaches that allow for examination of oligomeric Aβ. We showed that changing the ISF ApoE microenvironment in animals with established disease has striking and rapid allele specific effects on Aβ economy. Our study demonstrated that even a modest (∼10%) increase in ApoE4 levels, delivered to the ISF, markedly impacts the Aβ phenotype and clearance kinetics, with ApoE4 associated retention of increased soluble Aβ as well as fibrillar and formic acid extractable forms, and increases neurotoxicity around plaques marked by synaptic loss and increased neuritic dystrophies. Conversely, apoE2 decreases Aβ, and has a marked neuroprotective effect.

Since modest changes in the levels of ISF ApoE have such dramatic consequences, these results may lead to insight into the effects of a wide variety of environmental and genetic factors that might alter risk for AD or progression of AD by influencing *APOE* expression. Increases in ApoE of substantially more than the magnitude we demonstrated can occur after trauma, epilepsy, ischemia and high cholesterol diets, all of which have been associated with elevated cerebral Aβ. Moreover, promoter polymorphisms that have been previously found to be in genetic dysequilibrium with the *APOE4* allele impact *APOE* expression.

Other manipulations that impact ApoE or ApoE-lipoproteins homeostasis in the CNS clearly change Aβ deposition. For example, in experiments with focal gene transfer with APOE lentiviruses (primarily in hippocampal neurons), *APOE4* overexpression exerts a stronger effect on amyloid relative to *APOE3.* Previous studies also showed that RXR agonists, that have multiple effects including enhancing endogenous apoE synthesis, lead to clearance of Aβ from the brain perhaps by an effect on clearance across the blood brain barrier. In addition, brain transduction of CYP46A, which metabolizes cholesterol in the CNS and lowers its levels, reduces Aβ deposition as does increasing LDL-R in the brain, which is known to decrease apoE levels. Finally, genetic manipulations suggest that changing *apoE* expression by half can impact Aβ phenotype. Our results interestingly suggest that more modest changes can also have dramatic effects.

Our data directly address four other important areas of controversy in the APOE-Alzheimer literature. 1) We demonstrate a clear effect of ApoE isoform on neurotoxicity assessed by synapse loss and neuritic dystrophies, both likely related to impairments of neuronal system function. Since these effects were evident in the immediate vicinity of plaques, but not in areas distant from plaques, the synapse protective nature of ApoE2 compared to ApoE4 is likely mediated by effects on peri-plaque Aβ rather than due to a direct effect of ApoE on synaptic stability. 2) Direct observation of the kinetics of plaque deposition and growth using longitudinal multiphoton *in vivo* imaging show that ApoE4 enhances plaque deposition and growth, whereas ApoE2 is actually associated with resolution of plaques - arguing that the ApoE isoforms have a powerful impact on the pace and progression of disease beyond an initial effect on fibrillar plaque formation. The results reinforce the idea that ApoE4 may accelerate the disease process in terms of both amyloid deposition and neurotoxicity (and hence lead to an earlier age of onset) while ApoE2 does the opposite, which raises the possibility that introduction of ApoE2 (or an ApoE2 mimetic) into the CNS might have therapeutic value even after the disease is well established. 3) ApoE has variously been suggested as a mechanism to clear Aβ from the brain or as a retention molecule that increases clearance half-life; our current results show that introduction of modest amounts of ApoE into the ISF is sufficient to enhance retention of Aβ in the CNS, unless it is ApoE2. 4) The mechanisms of *APOE2*'s remarkable protective action in AD have long been unclear, in part since ApoE2 binds ApoE receptors relatively poorly. Our current data suggest that ApoE2 has a gain of function - able to actually reverse established Aβ deposits, as well as support synaptic and neuritic plasticity - in addition to a likely neutral or null effect on Aβ clearance into the plasma. This suggests that the decades long difference in age of onset between patients who inherit *APOE4* and *APOE2* alleles may reflect both a different initiation point and continuous differences in the kinetics of Aβ deposition and clearance as well as allele specific differences in the extent of neurotoxicity associated with the deposits. This dual function of ApoE2 may lead to therapeutic approaches aimed at mimicking its plaque clearing and synaptic restoration capacity.

These results are consistent with a model in which apoE acts as a scaffold for Aβ oligomerization, with efficiency of the formation and stabilization of oligomeric Aβ ApoE4>ApoE3>ApoE2, and with our recent observation that oligomeric Aβ is elevated in the CNS of ApoE4 >ApoE3 human patients with AD (even when plaque burden is normalized across cases). If ApoE, especially ApoE4, mediates formation of neurotoxic oligomeric Aβ, we predicted that enhanced ApoE4 would lead to increased synaptic and neuritic alterations, as appears to be the case in the current data. Based on these results, caution should be exercised with regard to agents that would increase ApoE levels in the brain in patients with AD who have inherited the *APOE4* allele.

Finally, our data confirm the power of AAV mediated transduction of ependyma to deliver secreted proteins to the brain and here, to the entire cortical mantle. Gene transfer or other approaches that decrease apoE4, or increase apoE2, are a powerful means of impacting AD disease progression.

### MATERIALS AND Methods

***Animals.*** Experiments were performed using both APPswe/PS1dE9 (APP/PS1) double transgenic mice (D. R. Borchelt et al., Accelerated amyloid deposition in the brains of transgenic mice coexpressing mutant presenilin 1 and amyloid precursor proteins. Neuron 19, 939 (Oct, 1997)) (obtained from Jackson laboratory, Bar Harbor, Maine) and Tg2576 mice (K. Hsiao et al., Correlative memory deficits, Abeta elevation, and amyloid plaques in transgenic mice. Science 274, 99 (Oct 4, 1996)). A human mutant amyloid precursor protein gene containing the Swedish double mutation K594N/M595L was inserted in the genome of these two mouse lines, under the control of the prion protein promoter. In addition, the APP/PS 1 mouse model overexpresses a variant of the Presenilin 1 gene deleted for the exon 9 (driven by the same promoter). The concomitant overexpression of APPswe and PSEN1 in APP/PS 1 mice leads to a more severe phenotype, with substantial amyloid deposition visible as soon as 6 months of age. On the other hand, the Tg2576 mouse line is a much milder model that only develops amyloid plaques around one year of age. To determine if the introduction of different ApoE isoforms would affect the progression of the disease, we respectively injected 7 months old and 16 months old APP/PS1 (between 4 and 7 animals per condition) and Tg2576 (between 3 to 5 animals per condition) mice. APOE-deficient mice (ApoE-KO, the Jackson Laboratory, Bar Harbor, Maine) were also used. Experiments were performed in accordance with NIH and institutional guidelines.

### Viral vectors construction and production

*APOE-2, -3* and *-4* cDNA were generously provided by Dr. LaDu at the University of Illinois (Chicago). After amplification by PCR, each of them was digested by BamHI and inserted into an AAV2-pCMV-hrGFP backbone. High titers of AAV serotype 4 vectors (AAV4-APOE2, AAV4-APOE3, AAV4-APOE4 and AAV4-GFP) were produced using the baculovirus system by the Gene Transfer Vector Core at the University of Iowa, Iowa City. Viruses were titered using quantitative PCR.

***Stereotactic Intraventricular injections.*** Stereotactic intraventricular injections of AAV serotype 4 vectors were performed as described previously (T. L. Spires et al., Dendritic spine abnormalities in amyloid precursor protein transgenic mice demonstrated by gene transfer and intravital multiphoton microscopy. J Neurosci 25, 7278 (Aug 3, 2005); G. Liu, I. H. Martins, J. A. Chiorini, B. L. Davidson, Adeno-associated virus type 4 (AAV4) targets ependyma and astrocytes in the subventricular zone and RMS. Gene Ther 12, 1503 (Oct, 2005)). Animals were anesthetized by intraperitoneal injection of ketamine/xylazine (100mg/kg and 50mg/kg body weight, respectively) and positioned on a stereotactic frame (David Kopf Instruments, Tujunga, CA). Injections of vectors were performed in each lateral ventricle with 5µl of viral preparation (titer 2 10 ¹² vg/ml) using a 33-gauge sharp micropipette attached to a 10-µl Hamilton syringe (Hamilton Medical, Reno, NV) at a rate of 0.25 µl/minute. Stereotactic coordinates of injection sites were calculated from bregma (anteroposterior +0.3 mm, mediolateral ± 1mm and dorsoventral -2mm).

***Cranial window implantation and multiphoton imaging.*** One week after intraventricular injection, mice were anesthesized with isoflurane (1.5%) and a cranial window was implanted by removing a piece of skull and replacing it with a glass coverslip of 8mm diameter (as described previously, T. L. Spires et al., Dendritic spine abnormalities in amyloid precursor protein transgenic mice demonstrated by gene transfer and intravital multiphoton microscopy. J Neurosci 25, 7278 (Aug 3, 2005)). For imaging, a wax ring was built along the border of the window to create a well of water for the objective (20 objective, numerical aperture of 0.95, Olympus). In order to visualize the amyloid deposits, transgenic animals received an intraperitoneal injection of methoxy-XO₄ (5mg/kg) 24hrs prior to surgery, a fluorescent compound that crosses the blood-brain barrier and binds to amyloid deposits (B. J. Bacskai, W. E. Klunk, C. A. Mathis, B. T. Hyman, Imaging amyloid-beta deposits in vivo. J Cereb Blood Flow Metab 22, 1035 (Sep, 2002)). Prior to imaging, Texas Red dextran (70,000 Da molecular weight; 12.5 mg/ml in sterile PBS; Molecular Probes, Eugene, OR) was injected into a lateral tail vein to provide a fluorescent angiogram, so that the shape of the vasculature would be used as a landmark to follow the exact same fields of view over time. Mice were imaged one week after AAV injection in order to evaluate the baseline level of amyloid deposits, then one and two month(s) after injection.

A mode-locked Ti:Sapphire laser (MaiTai, Spectra-Physics, Mountain View, CA) mounted on a multiphoton imaging system (Bio-Rad 1024ES, Bio-Rad, Hercules, CA) generated 860 nm two-photon fluorescence excitation light. Emitted light was collected through a custom-built external detector containing three photomultiplier tubes (Hamamatsu Photonics, Bridgewater, NJ), in the range of 380-480, 500-540 and 560-650 nm. 2-color images were acquired for plaques and angiography simultaneously. Low magnification *in vivo* images (615 615 µm; z-step, 2 µm, depth, ∼200 µm) were acquired and 6 to 8 fields of view were imaged to cover a large cortical area.

***Image processing and analysis.*** The density of plaque in each field of view was quantified using Image J by reporting the total number of amyloid deposits per volume of cortex imaged. We considered the cortical volume starting from the first slice of the z-stack at the surface to the last slice where an amyloid deposit could be detected. The size of amyloid deposits was evaluated over time by measuring their cross-sectional area from the maximal intensity after two-dimensional projection. For each plaque, the ratio of the area between the initial time point and the first month (T1/T0), or between the second and first months (T2/T1) was calculated.

The settings of the multiphoton microscope (laser power and PMTs) were maintained unchanged throughout the different imaging sessions during the whole time of the experiment.

***In vivo microdialysis sampling.** In vivo* microdialysis sampling of brain interstitial Aβ and ApoE was performed on Tg2576 mice, 3 months after intracerebroventricular injection of each AAV4 (S. Takeda et al., Novel microdialysis method to assess neuropeptides and large molecules in free-moving mouse. Neuroscience 186, 110 (Jul 14, 2011)). The microdialysis probe had a 4 mm shaft with a 3.0 mm, 1000 kDa molecular weight cutoff (MWCO) polyethylene (PE) membrane (PEP-4-03, Eicom, Kyoto, Japan). Before use, the probe was conditioned by briefly dipping it in ethanol, and then washed with artificial cerebrospinal fluid (aCSF) perfusion buffer (in mM: 122 NaCl, 1.3 CaCl2, 1.2 MgCl2, 3.0 KH2PO4, 25.0 NaHCO3) that was filtered through a 0.2-µm pore size membrane. The preconditioned probe's outlet and inlet were connected to a peristaltic pump (ERP-10, Eicom, Kyoto, Japan) and a microsyringe pump (ESP-32, Eicom, Kyoto, Japan), respectively, using fluorinated ethylene propylene (FEP) tubing (ϕ 250 µm i.d.).

Probe implantation was performed as previously described (S. Takeda et al., Novel microdialysis method to assess neuropeptides and large molecules in free-moving mouse. Neuroscience 186, 110 (Jul 14, 2011; J. R. Cirrito et al., In vivo assessment of brain interstitial fluid with microdialysis reveals plaque-associated changes in amyloid-beta metabolism and half-life. J Neurosci 23, 8844 (Oct 1, 2003)), with slight modifications. Briefly, anesthetized animals (1.5% isoflurane) were stereotactically implanted whit a guide cannula (PEG-4, Eicom, Kyoto, Japan) in the hippocampus (bregma -3.1 mm, -2.5 mm lateral to midline, -1.2 mm ventral to dura). The guide was then fixed to the skull using binary dental cement.

Four days after guide cannula implantation, the mice were placed in a standard microdialysis cage and a probe was inserted through the guide. After insertion of the probe, in order to obtain stable recordings, the probe and connecting tubes were perfused with aCSF for 240 min at a flow rate of 10 µl/min before sample collection. Samples were collected a flow rate of 0.25 (for Aβ quantification) and 0.1 µl/min (for ApoE detection). Samples were stored at 4°C in polypropylene tubes. During microdialysis sample collection, mice were awake and free-moving in the microdialysis cage designed to allow unrestricted movement of the animals without applying pressure on the probe assembly (AtmosLM microdialysis system, Eicom, Kyoto, Japan).

***Immunohistological analysis.*** APP/PS1 mice were euthanized by CO₂ inhalation 2 or 5 months after intraventricular injection (short and long term exposure), whereas Tg2576 animals were sacrificed after 3 months. One entire cerebral hemisphere was fixed in 4% paraformaldehyde in phosphate buffer saline for immunohistological analysis and embedded in paraffin wax. A 1mm coronal section through the frontal cortex was processed for the array tomography assay, whereas the rest of the hemibrain was snap frozen to perform biochemical and biomolecular analyses.

To detect amyloid deposits, ApoE and GFP, paraffin-embedded sections (10µm) were sequentially deparaffinized in xylene, rehydrated in ethanol, treated in citrate buffer (10mM Sodium Citrate, 0.05% Tween 20, pH 6.0), permeabilized in PBS with 0.5% Triton and blocked in PBS with 3% BSA for 2 hours at room temperature. Incubation with primary antibodies was done overnight at 4°C: Bam10 (SIGMA 1:1000) and R1282 (1:500, provided by Dr Dennis Selkoe) for amyloid plaques, mouse monoclonal antibody 3H1 (Ottawa Heart Institute) for human ApoE, Chicken anti-GFP (1:500, Aves) and SMI-312 (Covance) for neuritic dystrophies. Incubation with the secondary antibody was done for 2hrs at room temperature the next day. Amyloid dense core plaques were labeled by incubating the slices for 8 minutes in a solution of Thio-S (Sigma, St Louis, MO) 0.05% in 50% ethanol before mounting.

### Sample preparation, immunostaining and image analysis for array tomography

Array tomography analyses for pre- and post-synaptic elements were performed as previously described (R. M. Koffie et al., Oligomeric amyloid beta associates with postsynaptic densities and correlates with excitatory synapse loss near senile plaques. Proc Natl Acad Sci USA 106, 4012 (Mar 10, 2009)). Briefly, a piece of cortical tissue (1 mm³) adjacent to the ventricular region was dissected and fixed for 3h in 4% paraformaldehyde, 2.5% sucrose in 0.01M PBS. After dehydratation in ethanol, samples were incubated in LR White resin (Electron Microscopy Sciences) overnight at 4°C before polymerization at 53°C. Ribbons of sections (70nm) were then cut on an ultracut microtome (Leica) by using a Jumbo Histo Diamond Knife (Diatome).

After rehydration in 50 mM glycine in TBS for 5 minutes, sections were blocked in 0.05% Tween and 0.1% BSA in Tris for 5 minutes, and primary antibodies applied 1:50 in blocking buffer for 2 hours (PSD95 Abcam Ab12093, synapsin I Millipore AB1543 and NAB61 from Dr Virginia Lee that preferentially stains oligomeric Aβ species, E. B. Lee et al., Targeting amyloid-beta peptide (Abeta) oligomers by passive immunization with a conformation-selective monoclonal antibody improves learning and memory in Abeta precursor protein (APP) transgenic mice. J Biol Chem 281, 4292 (Feb 17, 2006)). Slides were washed with TBS and secondary antibodies applied (anti goat- Alexa Fluor 488, antimouse Cy3, or anti mouse Alexa Fluor 488 Invitrogen). Images were obtained on 7-30 serial sections through the frontal cortex and were acquired by using a Zeiss Axioplan LSM510 confocal/multiphoton microscope (63x numerical aperture Plan Apochromatic oil objective).

Images were analyzed as previously described using Image J (National Institutes of Health open software) and MATLAB (Mathworks) (R. M. Koffie et al., Oligomeric amyloid beta associates with postsynaptic densities and correlates with excitatory synapse loss near senile plaques. Proc Natl Acad Sci USA 106, 4012 (Mar 10, 2009)). Each set of images was converted to stacks, and aligned by using the Image J MultiStackReg and StackReg plug-ins (courtesy of Brad Busse and P. Thevenaz, Stanford University). Known volumes were selected and an automated, threshold-based detection program was used to count both PSD95 and synapsin puncta that appeared in more than one consecutive section (WaterShed program, provided by Brad Busse, Stephen Smith, and Kristina Micheva, Stanford University). Watershed exported a thresholded image stack (separate for each channel) showing puncta that were present in more than one slice of the array. Several sites in the cortex were sampled per mouse and their distance from the edge of a plaque was measured.

### Aβ quantification

The concentrations of Aβ₄₀ and Aβ₄₂ in the TBS soluble fraction, formic acid fraction as well as in the microdialysate were determined by BNT-77/BA-27 (for Aβ₄₀) and BNT-77/BC-05 (for Aβ₄₂) sandwich ELISA (Wako Pure Chemical Industries, Osaka, Japan), according to the manufacturer's instructions. The amount of oligomer Aβ in the sample was determined by 82E1/82E1 sandwich ELISA (Immuno-Biological Laboratories, Inc, Hamburg, Germany), in which the same N-terminal (residues 1-16) antibodies were used for both capture and detection (W. Xia et al., A specific enzyme-linked immunosorbent assay for measuring beta-amyloid protein oligomers in human plasma and brain tissue of patients with Alzheimer disease. Arch Neurol 66, 190 (Feb, 2009)).

### Immunoblot analysis

Brain TBS-soluble fractions and microdialysates (20 µg protein) were electrophoresed on 4-12% Novex Bis-Tris gels (Invitrogen) in MOPS running buffer for SDS-PAGE (Invitrogen). Gels were transferred to PVDF membrane, and blocked for 60 min at RT in 5% Milk / TBS-T. Membranes were probed with goat anti-ApoE antibody (1:1000, Millipore, AB947) to detect small amount of APOE in the ISF of APOE null animals, whereas albumin was detected as a control. Blots for human and mouse ApoE were respectively probed with EP1373Y antibody (1:1000, Novus Biologicals, NB110-55467) and with Rabbit polyclonal apoE antibody (1:1000, Abcam, ab20874). Incubation with HRP-conjugated goat IgG antibodies (Vector) was done for 2 hours. Immunoreactive proteins were developed using ECL kit (Western Lightning, PerkinElmer) and detected on Hyperfilm ECL (GE healthcare).

### qRT-PCR

Total RNA from brain samples were extracted using TRIzol® Reagent (Life technologies; 15596-026) and cDNA were then synthesized according to the SuperScript® III One-Step RT-PCR System (Life technologies; 12574-018) manufacturer instructions. PCR primers were specifically designed to amplify the recombinant human *APOE* mRNA and the endogenous *Apoe* and *Gapdh* mRNAs (*Apoe* Forward: 5'-AGCTCCCAAGTCACACAAGA ; *Apoe* Reverse : 5'- GTTGCGTAGATCCTCCATGT ; APOE Forward: 5'-CCAGCGACAATCACTGAAC ; APOE Reverse : 5'- GCGCGTAATACGACTCACTA; *Gapdh* Forward: 5'- ATGACATCAAGAAGGTGGTG and *Gapdh* Reverse: 5'-CATACCAGGAAATGAGCTTG).

### APOE ELISA

Specific ELISA assays were used to detected both human and endogenous murine APOE proteins. Briefly, ELISA plates were coated overnight with 1.5ug/ml of Goat anti-APOE antibody (to detect Murine APOE) or 1.5ug/ml WUE4 antibody (to detect Human APOE) and blocked with 1% non-fat milk diluted in PBS for 1.5h at 37°C. Human recombinant apoE proteins were used as standards (for human-specific assay, Biovision) or in-house mouse standards from brain extract (for the murine specific assay) and samples were diluted in ELISA buffer (0.5% BSA and 0.025% Tween-20 in PBS) and incubated overnight. After washing, detection antibodies specific for human (goat-apoe Millipore; 1:10,000) or mouse (Abcam ab20874 ; 1 :2,000) were respectively used, followed by 1.5h incubation with an appropriate HRP-conjugated secondary. Revelation of the signal was done using the TMB substrate before stopping the solution using H₃PO₄. The colorimetric results were measured at 450nm.

### Statistical analyses

Statistical analyses were performed using the Prism software. Because of the small size of the samples, normality could not be assumed for most of the analyses. For all the *post-mortem* analysis, a nonparametric Kruskal-Wallis test followed by a Dunn's Multiple Comparison Test was performed to evaluate the effect of each vector injected. *In vivo* imaging data of amyloid progression were analyzed using a mixed effects model, with a random effect for mouse, and fixed effects for vector, time and baseline volumetric density. An interaction between time and vector was considered in this analysis, but was not significant. For the analysis of the plaque size over time, two mixed effects models were fitted for log of the ratio of two consecutive time points, with random effects for mouse and fixed effects for log baseline size (t0 in the first analysis, t1 in the second analysis).

The use of the terms "a" and "an" and "the" and similar referents in the context of describing the invention are to be construed to cover both the singular and the plural, unless otherwise indicated herein or clearly contradicted by context. The terms "comprising," "having," "including," and "containing" are to be construed as open-ended terms (*i.e.,* meaning "including, but not limited to") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (*e.g*., "such as") provided herein, is intended merely to better illuminate the invention and does not pose a limitation on the scope of the invention unless otherwise claimed. No language in the specification should be construed as indicating any non-claimed element as essential to the practice of the invention.

Embodiments of this invention are described herein, including the best mode known to the inventors for carrying out the invention. Variations of those embodiments may become apparent to those of ordinary skill in the art upon reading the foregoing description.

## Claims

1. An rAAV2 particle comprising an AAV2 capsid protein and a vector comprising a nucleic acid encoding a protective ApoE ε2 isoform protein inserted between a pair of AAV2 inverted terminal repeats, for use in treating Alzheimer's disease in a mammal, wherein the rAAV particle is administered to an ependymal cell thereby delivering the nucleic acid to the ependymal cell, wherein the ependymal cell secretes the ApoE ε2 protein so as to treat the disease, and wherein the mammal is a non-rodent mammal.

2. The rAAV2 particle for use according to claim 1, wherein the rAAV2 particle is administered ex vivo to an ependymal cell from the non-rodent mammal and the ependymal cell is returned to the non-rodent mammal, thereby delivering the nucleic acid to the non-rodent mammal.

3. The rAAV2 particle for use according to claim 1, wherein the rAAV2 particle is administered to the cerebrospinal fluid (CSF) of the non-rodent mammal.

4. The rAAV2 particle for use according to any one of claims 1-3, wherein the non-rodent mammal is a primate, horse, sheep, goat, pig, or dog.

5. The rAAV2 particle for use according to any one of claims 1-3, wherein the non-rodent mammal is a human.

6. The rAAV2 particle for use according to any one of claims 1-5, wherein the vector further comprises a control element, wherein the control element directs the transcription or expression of the nucleic acid in the mammal in vivo.

7. A pharmaceutical composition comprising an rAAV2 particle comprising an AAV2 capsid protein and a vector comprising a nucleic acid encoding a protective ApoE ε2 isoform protein inserted between a pair of AAV2 inverted terminal repeats, for use in treating Alzheimer's disease in a mammal, wherein the rAAV2 particle is administered to an ependymal cell thereby delivering the nucleic acid to the ependymal cell, wherein the ependymal cell secretes the ApoE ε2 protein so as to treat the disease, and wherein the mammal is a non-rodent mammal.

## Patentansprüche

1. rAAV2-Partikel, umfassend ein AAV2-Kapsidprotein und einen Vektor, der eine Nukleinsäure, die für eine Isoform des schützenden Proteins ApoE ε2 kodiert, die zwischen einem Paar von invertierten terminalen Wiederholungen von AAV2 eingefügt ist, umfasst, zur Verwendung bei der Behandlung der Alzheimer-Krankheit in einem Säugetier, wobei der rAAV-Partikel an eine ependymale Zelle verabreicht wird, wodurch die Nukleinsäure an die ependymale Zelle abgegeben wird, wobei die ependymale Zelle das Protein ApoE ε2 sekretiert, um die Krankheit zu behandeln, und wobei das Säugetier kein Nagetier ist.

2. rAAV2-Partikel zur Verwendung gemäß Anspruch 1, wobei der rAAV2-Partikel ex vivo an eine ependymale Zelle aus dem Säugetier, das kein Nagetier ist, verabreicht wird und die ependymale Zelle an das Säugetier, das kein Nagetier ist, zurückgegeben wird, wodurch die Nukleinsäure an das Säugetier, das kein Nagetier ist, abgegeben wird.

3. rAAV2-Partikel zur Verwendung gemäß Anspruch 1, wobei der rAAV2-Partikel in die Cerebrospinalflüssigkeit (CSF) des Säugetiers, das kein Nagetier ist, verabreicht wird.

4. AAV2-Partikel zur Verwendung gemäß einem der Ansprüche 1-3, wobei das Säugetier, das kein Nagetier ist, ein Primat, Pferd, Schaf, Ziege, Schwein oder Hund ist.

5. AAV2-Partikel zur Verwendung gemäß einem der Ansprüche 1-3, wobei das Säugetier, das kein Nagetier ist, ein Mensch ist.

6. AAV2-Partikel zur Verwendung gemäß einem der Ansprüche 1-5, wobei der Vektor ferner ein Kontrollelement umfasst, wobei das Kontrollelement die Transkription oder Expression der Nukleinsäure in dem Säugetier in vivo steuert.

7. Pharmazeutische Zusammensetzung, umfassend einen rAAV2-Partikel, umfassend ein AAV2-Kapsidprotein und einen Vektor, der eine Nukleinsäure, die für eine Isoform des schützenden Protein ApoE ε2 kodiert, die zwischen einem Paar von invertierten terminalen Wiederholungen von AAV2 eingefügt ist, umfasst, zur Verwendung bei der Behandlung der Alzheimer-Krankheit in einem Säugetier, wobei der rAAV-Partikel an eine ependymale Zelle verabreicht wird, wodurch die Nukleinsäure an die ependymale Zelle abgegeben wird, wobei die ependymale Zelle das Protein ApoE ε2 sekretiert, um die Krankheit zu behandeln, und wobei das Säugetier kein Nagetier ist.

## Revendications

1. Particule de rAAV2 comprenant une protéine de capside d'AAV2 et un vecteur comprenant un acide nucléique codant pour une protéine isoforme ApoE ε2 protectrice insérée entre une paire de répétitions inversées terminales d'AAV2, pour une utilisation dans le traitement de la maladie d'Alzheimer chez un mammifère, dans laquelle la particule de rAAV est administrée à une cellule épendymaire pour ainsi délivrer l'acide nucléique à la cellule épendymaire, dans laquelle la cellule épendymaire sécrète la protéine ApoE ε2 afin de traiter la maladie, et dans laquelle le mammifère est un mammifère non-rongeur.

2. Particule de rAAV2 pour une utilisation selon la revendication 1, dans laquelle la particule de rAAV2 est administrée ex vivo à une cellule épendymaire provenant du mammifère non-rongeur et la cellule épendymaire est restituée au mammifère non-rongeur, pour ainsi délivrer l'acide nucléique au mammifère non-rongeur.

3. Particule de rAAV2 pour une utilisation selon la revendication 1, dans laquelle la particule de rAAV2 est administrée au liquide céphalorachidien (CSF) du mammifère non-rongeur.

4. Particule de rAAV2 pour une utilisation selon l'une quelconque des revendications 1 à 3, dans laquelle le mammifère non-rongeur est un primate, un cheval, un mouton, une chèvre, un cochon, ou un chien.

5. Particule de rAAV2 pour une utilisation selon l'une quelconque des revendications 1 à 3, dans laquelle le mammifère non-rongeur est un humain.

6. Particule de rAAV2 pour une utilisation selon l'une quelconque des revendications 1 à 5, dans laquelle le vecteur comprend en outre un élément de contrôle, dans laquelle l'élément de contrôle dirige la transcription ou l'expression de l'acide nucléique dans le mammifère in vivo.

7. Composition pharmaceutique comprenant une particule de rAAV2 comprenant une protéine de capside d'AAV2 et un vecteur comprenant un acide nucléique codant pour une protéine isoforme ApoE ε2 protectrice insérée entre une paire de répétitions inversées terminales d'AAV2, pour une utilisation dans le traitement de la maladie d'Alzheimer chez un mammifère, dans laquelle la particule de rAAV2 est administrée à une cellule épendymaire pour ainsi délivrer l'acide nucléique à la cellule épendymaire, dans laquelle la cellule épendymaire sécrète la protéine ApoE ε2 afin de traiter la maladie, et dans laquelle le mammifère est un mammifère non-rongeur.


### SEQUENCE LISTING

<110> UNIVERSITY OF IOWA RESEARCH FOUNDATION THE GENERAL HOSPITAL CORPORATION
<120> METHODS AND COMPOSITIONS FOR TREATING AMYLOID DEPOSITS
<130> 17023.122WO1
<140>
   <141>
<150> 61/648,801
   <151> 2012-05-18
<160> 10
<170> PatentIn version 3.5
<210> 1
   <211> 735
   <212> PRT
   <213> Adeno-associated virus 2
<400> 1
<210> 2
   <211> 734
   <212> PRT
   <213> Adeno-associated virus 4
<400> 2
   Met Thr Asp Gly Tyr Leu Pro Asp Trp Leu Glu Asp Asn Leu Ser Glu
   1               5                   10                  15
   Gly Val Arg Glu Trp Trp Ala Leu Gln Pro Gly Ala Pro Lys Pro Lys
               20                  25                  30
   Ala Asn Gln Gln His Gln Asp Asn Ala Arg Gly Leu Val Leu Pro Gly
           35                  40                  45
   Tyr Lys Tyr Leu Gly Pro Gly Asn Gly Leu Asp Lys Gly Glu Pro Val
       50                  55                  60
   Asn Ala Ala Asp Ala Ala Ala Leu Glu His Asp Lys Ala Tyr Asp Gln
   65                  70                  75                  80
   Gln Leu Lys Ala Gly Asp Asn Pro Tyr Leu Lys Tyr Asn His Ala Asp
                   85                  90                  95
   Ala Glu Phe Gln Gln Arg Leu Gln Gly Asp Thr Ser Phe Gly Gly Asn
               100                 105                 110
   Leu Gly Arg Ala Val Phe Gln Ala Lys Lys Arg Val Leu Glu Pro Leu
           115                 120                 125
   Gly Leu Val Glu Gln Ala Gly Glu Thr Ala Pro Gly Lys Lys Arg Pro
       130                 135                 140
   Leu Ile Glu Ser Pro Gln Gln Pro Asp Ser Ser Thr Gly Ile Gly Lys
   145 150 155 160
   Lys Gly Lys Gln Pro Ala Lys Lys Lys Leu Val Phe Glu Asp Glu Thr
   165 170 175
   Gly Ala Gly Asp Gly Pro Pro Glu Gly Ser Thr Ser Gly Ala Met Ser
   180 185 190
   Asp Asp Ser Glu Met Arg Ala Ala Ala Gly Gly Ala Ala Val Glu Gly
   195 200 205
   Gly Gln Gly Ala Asp Gly Val Gly Asn Ala Ser Gly Asp Trp His Cys
   210 215 220
   Asp Ser Thr Trp Ser Glu Gly His Val Thr Thr Thr Ser Thr Arg Thr
   225 230 235 240
   Trp Val Leu Pro Thr Tyr Asn Asn His Leu Tyr Lys Arg Leu Gly Glu
   245 250 255
   Ser Leu Gln Ser Asn Thr Tyr Asn Gly Phe Ser Thr Pro Trp Gly Tyr
   260 265 270
   Phe Asp Phe Asn Arg Phe His Cys His Phe Ser Pro Arg Asp Trp Gln
   275 280 285
   Arg Leu Ile Asn Asn Asn Trp Gly Met Arg Pro Lys Ala Met Arg Val
   290 295 300
   Lys Ile Phe Asn Ile Gln Val Lys Glu Val Thr Thr Ser Asn Gly Glu
   305 310 315 320
   Thr Thr Val Ala Asn Asn Leu Thr Ser Thr Val Gln Ile Phe Ala Asp
   325 330 335
   Ser Ser Tyr Glu Leu Pro Tyr Val Met Asp Ala Gly Gln Glu Gly Ser
   340 345 350
   Leu Pro Pro Phe Pro Asn Asp Val Phe Met Val Pro Gln Tyr Gly Tyr
   355 360 365
   Cys Gly Leu Val Thr Gly Asn Thr Ser Gln Gln Gln Thr Asp Arg Asn
   370 375 380
   Ala Phe Tyr Cys Leu Glu Tyr Phe Pro Ser Gln Met Leu Arg Thr Gly
   385 390 395 400
   Asn Asn Phe Glu Ile Thr Tyr Ser Phe Glu Lys Val Pro Phe His Ser
                   405                 410                 415
   Met Tyr Ala His Ser Gln Ser Leu Asp Arg Leu Met Asn Pro Leu Ile
               420                 425                 430
   Asp Gln Tyr Leu Trp Gly Leu Gln Ser Thr Thr Thr Gly Thr Thr Leu
           435                 440                 445
   Asn Ala Gly Thr Ala Thr Thr Asn Phe Thr Lys Leu Arg Pro Thr Asn
       450                 455                 460
   Phe Ser Asn Phe Lys Lys Asn Trp Leu Pro Gly Pro Ser Ile Lys Gln
   465                 470                 475                 480
   Gln Gly Phe Ser Lys Thr Ala Asn Gln Asn Tyr Lys Ile Pro Ala Thr
                   485                 490                 495
   Gly Ser Asp Ser Leu Ile Lys Tyr Glu Thr His Ser Thr Leu Asp Gly
               500                 505                 510
   Arg Trp Ser Ala Leu Thr Pro Gly Pro Pro Met Ala Thr Ala Gly Pro
           515                 520                 525
   Ala Asp Ser Lys Phe Ser Asn Ser Gln Leu Ile Phe Ala Gly Pro Lys
       530                 535                 540
   Gln Asn Gly Asn Thr Ala Thr Val Pro Gly Thr Leu Ile Phe Thr Ser
   545                 550                 555                 560
   Glu Glu Glu Leu Ala Ala Thr Asn Ala Thr Asp Thr Asp Met Trp Gly
                   565                 570                 575
   Asn Leu Pro Gly Gly Asp Gln Ser Asn Ser Asn Leu Pro Thr Val Asp
               580                 585                 590
   Arg Leu Thr Ala Leu Gly Ala Val Pro Gly Met Val Trp Gln Asn Arg
           595                 600                 605
   Asp Ile Tyr Tyr Gln Gly Pro Ile Trp Ala Lys Ile Pro His Thr Asp
       610                 615                 620
   Gly His Phe His Pro Ser Pro Leu Ile Gly Gly Phe Gly Leu Lys His
   625                 630                 635                 640
   Pro Pro Pro Gln Ile Phe Ile Lys Asn Thr Pro Val Pro Ala Asn Pro
                   645                 650                 655
   Ala Thr Thr Phe Ser Ser Thr Pro Val Asn Ser Phe Ile Thr Gln Tyr
   660 665 670
   Ser Thr Gly Gln Val Ser Val Gln Ile Asp Trp Glu Ile Gln Lys Glu
   675 680 685
   Arg Ser Lys Arg Trp Asn Pro Glu Val Gln Phe Thr Ser Asn Tyr Gly
   690 695 700
   Gln Gln Asn Ser Leu Leu Trp Ala Pro Asp Ala Ala Gly Lys Tyr Thr
   705 710 715 720
   Glu Pro Arg Ala Ile Gly Thr Arg Tyr Leu Thr His His Leu
   725 730
<210> 3
   <211> 2208
   <212> DNA
   <213> Adeno-associated virus 2
<400> 3
   atggctgccg atggttatct tccagattgg ctcgaggaca ctctctctga aggaataaga 60
   cagtggtgga agctcaaacc tggcccacca ccaccaaagc ccgcagagcg gcataaggac 120
   gacagcaggg gtcttgtgct tcctgggtac aagtacctcg gacccttcaa cggactcgac 180
   aagggagagc cggtcaacga ggcagacgcc gcggccctcg agcacgacaa agcctacgac 240
   cggcagctcg acagcggaga caacccgtac ctcaagtaca accacgccga cgcggagttt 300
   caggagcgcc ttaaagaaga tacgtctttt gggggcaacc tcggacgagc agtcttccag 360
   gcgaaaaaga gggttcttga acctctgggc ctggttgagg aacctgttaa gacggctccg 420
   ggaaaaaaga ggccggtaga gcactctcct gtggagccag actcctcctc gggaaccgga 480
   aaggcgggcc agcagcctgc aagaaaaaga ttgaattttg gtcagactgg agacgcagac 540
   tcagtacctg acccccagcc tctcggacag ccaccagcag ccccctctgg tctgggaact 600
   aatacgatgg ctacaggcag tggcgcacca atggcagaca ataacgaggg cgccgacgga 660
   gtgggtaatt cctcgggaaa ttggcattgc gattccacat ggatgggcga cagagtcatc 720
   accaccagca cccgaacctg ggccctgccc acctacaaca accacctcta caaacaaatt 780
   tccagccaat caggagcctc gaacgacaat cactactttg gctacagcac cccttggggg 840
   tattttgact tcaacagatt ccactgccac ttttcaccac gtgactggca aagactcatc 900
   aacaacaact ggggattccg acccaagaga ctcaacttca agctctttaa cattcaagtc 960
   aaagaggtca cgcagaatga cggtacgacg acgattgcca ataaccttac cagcacggtt 1020
   caggtgttta ctgactcgga gtaccagctc ccgtacgtcc tcggctcggc gcatcaagga 1080
   tgcctcccgc cgttcccagc agacgtcttc atggtgccac agtatggata cctcaccctg 1140
   aacaacggga gtcaggcagt aggacgctct tcattttact gcctggagta ctttccttct 1200
   cagatgctgc gtaccggaaa caactttacc ttcagctaca cttttgagga cgttcctttc 1260
   cacagcagct acgctcacag ccagagtctg gaccgtctca tgaatcctct catcgaccag 1320
   tacctgtatt acttgagcag aacaaacact ccaagtggaa ccaccacgca gtcaaggctt 1380
   cagttttctc aggccggagc gagtgacatt cgggaccagt ctaggaactg gcttcctgga 1440
   ccctgttacc gccagcagcg agtatcaaag acatctgcgg ataacaacaa cagtgaatac 1500
   tcgtggactg gagctaccaa gtaccacctc aatggcagag actctctggt gaatccgggc 1560
   ccggccatgg caagccacaa ggacgatgaa gaaaagtttt ttcctcagag cggggttctc 1620
   atctttggga agcaaggctc agagaaaaca aatgtggaca ttgaaaaggt catgattaca 1680
   gacgaagagg aaatcaggac aaccaatccc gtggctacgg agcagtatgg ttctgtatct 1740
   accaacctcc agagaggcaa cagacaagca gctaccgcag atgtcaacac acaaggcgtt 1800
   cttccaggca tggtctggca ggacagagat gtgtaccttc aggggcccat ctgggcaaag 1860
   attccacaca cggacggaca ttttcacccc tctcccctca tgggtggatt cggacttaaa 1920
   caccctcctc cacagattct catcaagaac accccggtac ctgcgaatcc ttcgaccacc 1980
   ttcagtgcgg caaagtttgc ttccttcatc acacagtact ccacgggaca ggtcagcgtg 2040
   gagatcgagt gggagctgca gaaggaaaac agcaaacgct ggaatcccga aattcagtac 2100
   acttccaact acaacaagtc tgttaatgtg gactttactg tggacactaa tggcgtgtat 2160
   tcagagcctc gccccattgg caccagatac ctgactcgta atctgtaa 2208
<210> 4
   <211> 2205
   <212> DNA
   <213> Adeno-associated virus 4
<400> 4
   atgactgacg gttaccttcc agattggcta gaggacaacc tctctgaagg cgttcgagag      60
   tggtgggcgc tgcaacctgg agcccctaaa cccaaggcaa atcaacaaca tcaggacaac     120
   gctcggggtc ttgtgcttcc gggttacaaa tacctcggac ccggcaacgg actcgacaag     180
   ggggaacccg tcaacgcagc ggacgcggca gccctcgagc acgacaaggc ctacgaccag     240
   cagctcaagg ccggtgacaa cccctacctc aagtacaacc acgccgacgc ggagttccag     300
   cagcggcttc agggcgacac atcgtttggg ggcaacctcg gcagagcagt cttccaggcc     360
   aaaaagaggg ttcttgaacc tcttggtctg gttgagcaag cgggtgagac ggctcctgga     420
   aagaagagac cgttgattga atccccccag cagcccgact cctccacggg tatcggcaaa     480
   aaaggcaagc agccggctaa aaagaagctc gttttcgaag acgaaactgg agcaggcgac     540
   ggaccccctg agggatcaac ttccggagcc atgtctgatg acagtgagat gcgtgcagca 600
   gctggcggag ctgcagtcga gggcggacaa ggtgccgatg gagtgggtaa tgcctcgggt      660
   gattggcatt gcgattccac ctggtctgag ggccacgtca cgaccaccag caccagaacc      720
   tgggtcttgc ccacctacaa caaccacctc tacaagcgac tcggagagag cctgcagtcc 780
   aacacctaca acggattctc caccccctgg ggatactttg acttcaaccg cttccactgc 840
   cacttctcac cacgtgactg gcagcgactc atcaacaaca actggggcat gcgacccaaa      900
   gccatgcggg tcaaaatctt caacatccag gtcaaggagg tcacgacgtc gaacggcgag      960
   acaacggtgg ctaataacct taccagcacg gttcagatct ttgcggactc gtcgtacgaa     1020
   ctgccgtacg tgatggatgc gggtcaagag ggcagcctgc ctccttttcc caacgacgtc     1080
   tttatggtgc cccagtacgg ctactgtgga ctggtgaccg gcaacacttc gcagcaacag     1140
   actgacagaa atgccttcta ctgcctggag tactttcctt cgcagatgct gcggactggc     1200
   aacaactttg aaattacgta cagttttgag aaggtgcctt tccactcgat gtacgcgcac     1260
   agccagagcc tggaccggct gatgaaccct ctcatcgacc agtacctgtg gggactgcaa      1320
   tcgaccacca ccggaaccac cctgaatgcc gggactgcca ccaccaactt taccaagctg     1380
   cggcctacca acttttccaa ctttaaaaag aactggctgc ccgggccttc aatcaagcag     1440
   cagggcttct caaagactgc caatcaaaac tacaagatcc ctgccaccgg gtcagacagt     1500
   ctcatcaaat acgagacgca cagcactctg gacggaagat ggagtgccct gacccccgga     1560
   cctccaatgg ccacggctgg acctgcggac agcaagttca gcaacagcca gctcatcttt     1620
   gcggggccta aacagaacgg caacacggcc accgtacccg ggactctgat cttcacctct     1680
   gaggaggagc tggcagccac caacgccacc gatacggaca tgtggggcaa cctacctggc     1740
   ggtgaccaga gcaacagcaa cctgccgacc gtggacagac tgacagcctt gggagccgtg     1800
   cctggaatgg tctggcaaaa cagagacatt tactaccagg gtcccatttg ggccaagatt     1860
   cctcataccg atggacactt tcacccctca ccgctgattg gtgggtttgg gctgaaacac     1920
   ccgcctcctc aaatttttat caagaacacc ccggtacctg cgaatcctgc aacgaccttc     1980
   agctctactc cggtaaactc cttcattact cagtacagca ctggccaggt gtcggtgcag     2040
   attgactggg agatccagaa ggagcggtcc aaacgctgga accccgaggt ccagtttacc     2100
   tccaactacg gacagcaaaa ctctctgttg tgggctcccg atgcggctgg gaaatacact     2160
   gagcctaggg ctatcggtac ccgctacctc acccaccacc tgtaa                     2205
<210> 5
   <211> 20
   <212> DNA
   <213> Artificial Sequence
<220>
   <223> Description of Artificial Sequence: Synthetic primer
<400> 5
   agctcccaag tcacacaaga 20
<210> 6
   <211> 20
   <212> DNA
   <213> Artificial Sequence
<220>
   <223> Description of Artificial Sequence: Synthetic primer
<400> 6
   gttgcgtaga tcctccatgt 20
<210> 7
   <211> 19
   <212> DNA
   <213> Artificial Sequence
<220>
   <223> Description of Artificial Sequence: Synthetic primer
<400> 7
   ccagcgacaa tcactgaac 19
<210> 8
   <211> 20
   <212> DNA
   <213> Artificial Sequence
<220>
   <223> Description of Artificial Sequence: Synthetic primer
<400> 8
   gcgcgtaata cgactcacta 20
<210> 9
   <211> 20
   <212> DNA
   <213> Artificial Sequence
<220>
   <223> Description of Artificial Sequence: Synthetic primer
<400> 9
   atgacatcaa gaaggtggtg 20
<210> 10
   <211> 20
   <212> DNA
   <213> Artificial Sequence
<220>
   <223> Description of Artificial Sequence: Synthetic primer
<400> 10
   cataccagga aatgagcttg 20